(19) Europäisches Patentamt
European Patent Office
Office européen des brevets

(11) **EP 2 504 061 B1**

(12) **EUROPÄISCHE PATENTSCHRIFT**

(45) Veröffentlichungstag und Bekanntmachung des Hinweises auf die Patenterteilung:
**29.01.2014 Patentblatt 2014/05**

(21) Anmeldenummer: **10752535.4**

(22) Anmeldetag: **31.08.2010**

(51) Int Cl.:
***A61N 5/10*** *(2006.01)*

(86) Internationale Anmeldenummer:
**PCT/EP2010/062737**

(87) Internationale Veröffentlichungsnummer:
**WO 2011/064004 (03.06.2011 Gazette 2011/22)**

(54) **VERFAHREN UND VORRICHTUNG ZUR STEUERUNG DER DOSISAPPLIKATION BEI DER BESTRAHLUNG**

METHOD AND DEVICE FOR CONTROLLING DOSAGE APPLICATION DURING IRRADIATION

PROCÉDÉ ET DISPOSITIF POUR COMMANDER L'APPLICATION DE DOSE LORS DE L'IRRADIATION

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **26.11.2009 DE 102009055902**

(43) Veröffentlichungstag der Anmeldung:
**03.10.2012 Patentblatt 2012/40**

(73) Patentinhaber: **GSI Helmholtzzentrum für Schwerionenforschung GmbH
64291 Darmstadt (DE)**

(72) Erfinder:
• **BERT, Christoph
63741 Aschaffenburg (DE)**
• **LÜCHTENBORG, Robert
64827 Darmstadt (DE)**

(74) Vertreter: **Rück, Dorothee Maria
GSI
Helmholtzzentrum für
Schwerionenforschung GmbH
Planckstraße 1
D-64291 Darmstadt (DE)**

(56) Entgegenhaltungen:
**DE-A1-102007 045 879**

• **BERT CHRISTOPH ET AL: "4D treatment planning for scanned ion beams" RADIATION ONCOLOGY, BIOMED CENTRAL LTD, LO LNKD-DOI:10.1186/1748-717X-2-24, Bd. 2, Nr. 1, 3. Juli 2007 (2007-07-03), Seite 24, XP021030775 ISSN: 1748-717X**

**Beschreibung**

Gebiet der Erfindung

Die Erfindung betrifft ein Verfahren zur Steuerung der Dosisapplikation bei der Bestrahlung eines beweglichen Zielvolumens in einem Körper mit einem energetischen Strahl, insbesondere mit einem nadelfeinen Ionenstrahl mit welchem das Zielvolumen abgerastert wird, sowie eine entsprechende Bestrahlungsvorrichtung.

Hintergrund der Erfindung

Bei der Behandlung von Tumoren im Allgemeinen kommen operative Resektionen, Strahlen- und Chemotherapie zum Einsatz oder eine Kombination aus diesen Verfahren. Bei der Strahlentherapie ist es das Ziel der Behandlung, eine hohe lokale Tumordosis bei möglichst minimaler Belastung des umliegenden Normalgewebes zu erzielen. Hierzu wird die Energie- bzw. Dosisdeposition der Strahlung möglichst konform an den Tumor angepasst. In letzter Zeit werden gute Therapieerfolge mit Bestrahlung durch Ionen statt Photonen erzielt, da die Energie- bzw. Dosisdeposition als Funktion der Eindringtiefe ein scharfes Maximum (sogenannter Bragg-Peak) aufweist. Ein bekanntes Verfahren ist die passive Strahlapplikation, bei welcher der Strahl durch einen speziell angepassten Kollimator geformt wird. Alternativ ist es jedoch sogar möglich, den Ionenstrahl präzise zu fokussieren und den Tumor mit einem nadelfeinen Strahl, einem sogenannten "Pencil-Beam" dreidimensional abzuscannen

(Raster-Scan-Verfahren, Spot-Scan-Verfahren, Continous-Scan-Verfahren). Beim Raster-Scan-Verfahren verbleibt der Strahl für eine definierte Zeit auf einer Rasterposition und bleibt beim Wechsel zur nächsten Rasterposition angeschaltet. Beim Spot-Scan-Verfahren wird der Strahl im Gegensatz dazu zwischen den Rasterpositionen abgeschaltet. Beim Continous-Scan-Verfahren wird der Strahl, ohne stationär auf den Rasterpositionen zu verweilen, stetig kontinuierlich über diese gefahren.

Neben Protonen werden derzeit auch andere Ionen, insbesondere Kohlenstoff-Ionen verwendet. Teilweise werden auch Neon-Ionen verwendet. Der Einsatz dieser Ionen zeichnet sich durch eine erhöhte relative biologische Wirksamkeit (RBW) gegenüber Photonen bei der Inaktivierung von Zellen aus. Aufgrund ihrer Abhängigkeit vom Dosislevel, dem Gewebetyp und vor allem der Teilchensorte und -energie ist die RBW im tiefliegenden Tumorbereich in der Regel höher als im Eingangskanal und führt damit zu einem zusätzlichen therapeutischen Nutzen.

In den letzten Jahren wurde ein großer klinischer Erfolg durch die Anmelderin in Zusammenarbeit mit der Universität Heidelberg, dem Deutschen Krebsforschungszentrum und dem Forschungszentrum Dresden/Rossendorf mit Bestrahlungen im Raster-Scan-Verfahren mit Kohlenstoffionen und dedizierter Bestrahlungsplanung erzielt. Vorteile dieses Verfahrens sind der weitgehende Verzicht auf Absorbermaterialien zur Vermeidung der Erzeugung von Sekundärteilchen und vor allem die gute Konformität der erzeugten Dosisverteilungen, im Vergleich zur passiven Strahlapplikation insbesondere proximal des Tumors.

Anfangs wurden hauptsächlich Tumore im Schädelbasisbereich und entlang der Wirbelsäule behandelt, deren Bewegung man durch stereotaktische Fixierung auf ein vernachlässigbares Minimum reduzieren kann. Mit der geplanten breiteren klinischen Anwendung des Raster-Scan-Verfahrens in diversen Therapiezentren sollen jedoch auch andere Tumoren mit Ionenstrahlen, insbesondere Kohlenstoffionen-Strahlen im Raster-Scan-Verfahren bestrahlt werden. Tumoren im Rumpfbereich des Körpers unterliegen jedoch einer stärkeren Bewegung, beispielsweise durch die Atmung des Patienten, bei der sich der gesamte Brustkorb und Teile des Abdomens bewegen und verformen, ggf. sogar durch den Herzschlag des Patienten. Bei der Behandlung bewegter Tumoren oder allgemein bewegter Zielvolumina mit einem Scan-Verfahren steht man vor der Herausforderung, dass sich diese Bewegung ungünstig auf die Homogenität der Energiedeposition auswirken kann. Experimente mit Phantomen haben gezeigt, dass bei der Applikation eines gescannten Strahls Über- und Unterdosierungen im Zielvolumen auftreten können, so dass eine einfache Erweiterung des Zielvolumens um das Ausmaß der Bewegung, wie sie bei passiver Strahlapplikation eingesetzt wird, keine optimale Behandlung gewährleistet.

Um den Einfluss der Bewegung bei gescannter Strahlapplikation zu korrigieren, werden derzeit entweder die oft fraktionierte Bestrahlung unter Verwendung von Sicherheitssäumen, Mehrfachbestrahlung (sogenanntes "Rescanning"), in Abhängigkeit der Bewegungsphase unterbrochene Bestrahlung (sogenanntes "Gating"), bewegungskompensierte Bestrahlung mit aktiver Strahlnachführung (sogenanntes "Tracking") oder Kombinationen der genannten Methoden/Verfahren untersucht und in prä-klinischen Untersuchungen eingesetzt. Bei der bewegungskompensierten Bestrahlung mit aktiver Strahlnachführung (Tracking) wird die Strahlposition fortwährend an die Tumorbewegung angepasst. Hierbei werden die Strahllage lateral zur Strahlrichtung und die Teilchenreichweite kontinuierlich an die Tumorbewegung angepasst. Diesbezüglich wird auf die Dissertationen von S.O. Grötzinger, "Volume Conformal Irradiation of Moving Target Volumes with scanned ion beams", TU Darmstadt, 2004 und C. Bert "Bestrahlungsplanung für bewegte Zielvolumina in der Tumortherapie mit gescanntem Kohlenstoffstrahl", TU Darmstadt, 2006 verwiesen. Jedenfalls ist die bewegungskompensierte Raster-Scan-Ionenstrahlapplikation dem Fachmann auf dem Gebiet der Partikelstrahl-Tumortherapie grundsätzlich bekannt.

Die Therapie von sich bewegenden Tumoren mit einem gescannten Teilchenstrahl kann also ohne Verwendung von Gegenmaßnahmen ("motion mitigation") grundsätzlich zu Fehldosierungen führen, die auf die Wechselwirkung von dynamischer Bestrahlung und bewegter Anatomie zurückzuführen sind. Selbst wenn man die vorstehend genannten Verfahren, wie z.B. die aktive Strahlnachführung (Tracking) anwendet, kann sich trotz Strahlnachführung der Weg des Teilchenstrahls im Gewebe verändern, z.B. wenn die Tumorbewegung nicht durch eine reine Translation beschrieben werden kann. Dies ist sogar häufig der Fall, da eine Bewegung, z.B. des Brustkorbes, Rotationsanteile und/oder Deformationen des Gewebes enthalten kann. Während durch die aktive Strahlnachführung (Tracking) die Bragg-Maximum-Position des Teilchenstrahls und damit der Großteil der Teilchendosis an die anatomisch korrekte Position gelenkt wird, verändert sich dennoch auf Grund des veränderten Strahlweges der Dosisbeitrag zum übrigen Gewebe, vor allem proximal, d.h. strahlaufwärts der aktuellen Rasterposition in der das Bragg-Maximum sitzt. Dies führt zu lokalen Unter- und Überdosierungen im Vergleich zur geplanten Dosisdeposition, was nachteilig sein kann. Die Erfinder haben in simulierten Behandlungen basierend auf gemessenen Lungentumordaten herausgefunden, dass die Dosisabdeckung ohne Berücksichtigung der beschriebenen Dosisänderungen im Vergleich zur simulierten Bestrahlung eines hypothetisch unbewegten Lungentumors (statische Bestrahlung, hier treten keine Dosisänderungen auf) deutlich schlechter ist.

Die Erfindung sucht insbesondere diese Probleme zu lösen.

In der DE 10 2005 063 220 A1 werden Maßnahmen zur Verbesserung des zeitlichen Ablaufs einer Bestrahlung beschrieben, die in Bezug auf die vorstehend genannten Probleme jedoch weiter verbesserungsfähig sind.

Auch Dokumente DE 10 2007 045879 sowie "4D treatment planning for scanned ion beams" (Bert C., Rietzel E., Radiat. Oncol. 2007, 2:24) beschreiben ähnliche System und Verfahren.

Allgemeine Beschreibung der Erfindung

Die Erfindung hat sich daher die Aufgabe gestellt, ein Verfahren und eine Vorrichtung zur Dosisapplikation bereit zu stellen, welche die Qualität der Bestrahlung eines bewegten Zielvolumens verbessern, insbesondere trotz Bewegung eine gute Übereinstimmung der Ist-Dosisverteilung bei der Bestrahlung mit einer vorher festgelegten Soll-Dosisverteilung zu erreichen.

Die Erfindung ist in den Ansprüchen definiert.

Eine weitere Aufgabe der Erfindung ist es, ein Verfahren und eine Vorrichtung zur Dosisapplikation bereit zu stellen, welche den Bestrahlungsablauf optimieren.

Die Aufgabe der Erfindung wird durch den Gegenstand der unabhängigen Ansprüche gelöst. Vorteilhafte Weiterbildungen der Erfindung sind in den Unteransprüchen definiert.

Erfindungsgemäß wird ein bewegliches Zielvolumens in einem Körper mit einem energetischen Strahl mit einem Abtast-Verfahren bestrahlt. Der Körper kann z.B. ein lebender menschlicher oder tierischer Körper sein, der im Rahmen einer Tumorbehandlung bestrahlt wird, kann aber auch ein Modell (Tiermodell, Zellkultur, sonstiges Phantom) zur Validierung einer Patientenbestrahlung oder ein anderer nichtlebender Körper sein. Der Strahl ist insbesondere ein Partikelstrahl, vorzugsweise ein Ionenstrahl, z.B. ein hochenergetischer Kohlenstoffionen-Strahl. Die vorliegende Erfindung betrifft dabei die Steuerung der Bestrahlungsvorrichtung, z.B. unter dem Gesichtspunkt der Qualität und Präzision der Strahlapplikation, aber auch der benötigten Rechenleistung trotz Bewegung des Zielvolumens. Erfindungsgemäß wird ein Abtastverfahren eingesetzt, bei welchem das Zielvolumen dreidimensional in eine Vielzahl von Rasterpositionen unterteilt wird und diese Rasterpositionen nacheinander mit einem präzise fokussierten nadelfeinen Ionenstrahl abgetastet oder abgerastert werden. Dem Fachmann ist dieses Verfahren als Scan-Verfahren bekannt. Wie vorstehend beschrieben wurde, erhalten zwar tatsächlich immer mehrere Rasterpositionen, z.B. die im Strahlenweg liegenden Rasterpositionen eine gewisse Strahlendosis, es ist dem Fachmann jedoch ersichtlich, dass mit der Bezeichnung Bestrahlung der $i$-ten Rasterposition diejenige Position gemeint ist, in welche das Bragg-Maximum gesetzt ist, d.h. diejenige Rasterposition, die die größte Dosis erhält. Die Steuereinrichtung für die Beschleunigeranlage steuert den Ionenstrahl also bei dem Scan-Verfahren derart, dass die einzelnen Rasterpositionen mit dem Ionenstrahl mit rasterpositionsabhängig gesteuerter Teilchenenergie und Teilchenfluenz (Teilchenanzahl pro Flächeneinheit) nacheinander abgetastet werden, so dass eine rasterpositionsabhängige Dosis an der jeweiligen Rasterposition appliziert wird. Die vorliegende Erfindung eignet sich insbesondere für die in der Einleitung näher erläuterten Raster-Scan-Verfahren und Spot-Scan-Verfahren.

Bei diesen Scan-Verfahren wird zunächst im Vorfeld der Bestrahlung mit einem zeitauflösenden dreidimensional bildgebenden Diagnosesystem, z.B. einem zeitauflösenden dreidimensionalen Computertomographen (sogenanntes 4D-CT), einem zeitauflösenden dreidimensionalen Magnetresonanztomographen (sogenanntes 4D-MRT) oder einem zeitauflösenden dreidimensionalen Positronen Emmissions Tomographen (sogenanntes 4D-PET) die zeitaufgelöste Struktur des zu bestrahlenden Körpers und insbesondere des Zielvolumens, z.B. Tumors unter dem Einfluss der Bewegung des Körpers, z.B. aufgrund der Atmung aufgezeichnet. Hierbei wird z.B. wie folgt vorgegangen:

1) Aufnahme eines 4D-CTs (d.h. mehrere 3D-CT (Bewegungsphasen M) die durch zeitliche Korrelation mit einem Bewegungssurrogat zeitlich im Atemzyklus geordnet werden)

2) Ggf. Aufnahme eines 4D-MRT/4D-PET (Segmentierung, Staging)

3) Definition einer Referenzbewegungsphase, insbesondere bei Ausatmung

4) Registrierung der M-1 Bewegungsphasen zur Referenzbewegungsphase durch (nicht-rigide) Transformationen (Optimierungsvorgang bei dem z.B. die normierte wechselseitige Information, sogenannte "normalized mutual information" minimiert wird). Die entstehenden M-1 Transformationen (und deren Inverse) können die 3D Bewegung des Tumors beschreiben

5) Optimierung eines quasi-stationären Referenzbestrahlungsplans unter Verwendung der Referenzphase des 4D-CT (die 3-dimensional ist) und den segmentierten Volumina (Tumor und Risiko-Organ, sogenanntes "organ at risk", OAR) aus dem (4D)-MR oder dem Kontrastmittel-CT oder 4D-CT. Dies ist dem Fachmann grundsätzlich bekannt, z.B. aus Krämer et al. "Treatment planning for heavy-ion radiotherapy: physical beam model and dose optimization. Phys Med Biol 2000, 45:33299-3317, Krämer et al. "Treatment planning for heavy-ion radiotherapy: calculation and optimization of biologically effective dose. Phys Med Biol 2000, 45:3319-3330, Jäkel and Krämer, "Treatment planning for heavy ion irradiation", Phys. Med. 14 53-62

6) Kombination von Referenzbestrahlungsplan, Transformationsparametern und 4D-CT Phasen, um für alle Rasterpunkte und alle Phasen Adaptionsparameter dx, dy, dE zu bestimmen

7) Bestimmung der Dosisbeiträge von Rasterpunkt i zu Rasterpunkt k in Abhängigkeit von der Bewegungsphase m, nachfolgend $D_m^{ik}$ genannt

8) Beginn der Bestrahlung, mit dem nachfolgend noch detailliert erläuterten Bestrahlungsverfahren

Im Vorfeld der Bestrahlung wird mithilfe von einer Bestrahlungsplanungseinrichtung, welche einen Computer umfasst, der sogenannte Bestrahlungsplan erstellt, wie dem Fachmann bekannt ist. Hierzu wird, aufgelöst nach den Bewegungsphasen, für einen oder mehrere Bestrahlungsdurchläufe für jede Rasterposition ein Datentupel angelegt, welches zumindest vier Elemente umfasst, nämlich $x$-Position, $y$-Position, Teilchenenergie und Teilchenfluenz enthält. Aufgrund der Vielzahl der Rasterpositionen und der Komplexität des Problems kann die Erstellung relativ lange dauern, so dass der Bestrahlungsplan typischerweise vollständig, ggf. Stunden oder Tage vor der Bestrahlung erstellt und abgespeichert wird. Bei den bisher bekannten Verfahren wurde der Patient dann üblicherweise mit den Werten aus dem vorher festgelegten Bestrahlungsplan bestrahlt.

Anhand der 4D Daten können durch dem Fachmann bekannte Registrierungsalgorithmen (nicht-rigide) Transformationen, d.h. Bewegungsdaten, zwischen den einzelnen Bewegungsphasen ermittelt werden (vgl. vorstehend unter Ziffer 4). Mit anderen Worten wird anhand der 4DCT Bewegungsphasen eine Mehrzahl von Zeitintervallen definiert, in welchen die Transformation der Parameter in die Referenzbewegungsphase ermittelt und in Form von Positionsadaptionsparametern gespeichert wird. Die Zeitintervalle können hierbei mit den durch die (M-1) Bewegungsphasen definierten Intervallen identisch sein. Es ist ebenfalls denkbar zusätzliche Zeitinvervalle einzuführen. Die Bewegungsphasen ergeben sich also aus den 4D-CT/4D-MRT-Daten. Die Registrierung optimiert dann eine Transformation, um z.B. von Ausatmung in die Einatmung zu kommen.

Anhand dieser Daten kann später bei der Bestrahlung in Echtzeit anhand einer Bewegungsmessung am Körper bestimmt werden, welche Rasterposition des Zielvolumens das Bragg-Maximum unter Bewegungseinfluss tatsächlich trifft. Die zur Verfügung gestellten Kompensationsparameter, erlauben es dann, die "richtige" Position zu treffen.

Erfindungsgemäß wird nun zunächst an der Erstellung des Bestrahlungsplans im Vorfeld der Bestrahlung festgehalten, wobei der Datensatz aus den Datentupeln einem Referenz-Datensatz entspricht, in welchem die Teilchenfluenz des Bestrahlungsplans erfindungsgemäß einer nominellen Teilchenfluenz entspricht, welche während der Bestrahlung in Echtzeit in Abhängigkeit des Bestrahlungsverlaufs noch abgeändert wird. Es wird also ein Bestrahlungsplan mit einer Vielzahl von Rasterpositionen $i$, $1<=i<=N$ des Zielvolumens erstellt und abgespeichert, welcher für die Bestrahlung der Rasterpositionen $i$ jeweils einen Referenz-Datensatz mit den Positionsdaten der jeweiligen Rasterposition und einer rasterpositionsabhängigen nominellen Teilchenfluenz enthält.

Mit einer Bewegungserfassungseinrichtung wird während der Bestrahlung die Bewegung des Körpers fortwährend erfasst, um festzustellen, welche Bewegungsphase zu jedem Zeitpunkt der Bestrahlung vorliegt. Hieraus kann die tatsächliche Bewegung der Rasterpositionen fortwährend und 3-dimensional während der Bestrahlung anhand der, z.B. aus der 4D-CT oder 4D-MRT-Untersuchung gewonnenen, Positionsadaptionsparameter ermittelt werden.

Die Erfindung umfasst hierzu eine Recheneinrichtung, welche eingerichtet ist, die vor der Bestrahlung abgespeicherten Datensätze des Bestrahlungsplans während der Bestrahlung auszulesen und in Echtzeit zu ändern, um unmittelbar anschließend mit den geänderten Werte, insbesondere einer geänderten Teilchenfluenz zu bestrahlen.

Typischerweise umfasst ein Bestrahlungsplan $N$ Rasterpositionen, wobei die Anzahl $N$ einige tausend bis einige zehntausend oder sogar noch mehr betragen kann. Wie im Folgenden noch näher erläutert wird, wird erfindungsgemäß für jede $i$-te Rasterposition $1<=i<=N$ die tatsächlich deponierte Teilchenfluenz, welche die $i$-te Rasterposition bei der Bestrahlung der anderen vorher bestrahlten $k$-ten Rasterposition $1<=k<i$ in der während der Bestrahlung der Rasterposition $k$ ermittelten Bewegungsphase $m$ erhalten hat, ermittelt und weiterverarbeitet. Um diese Berechnungen online bzw. in Echtzeit durchführen zu können, werden erfindungsgemäß für jede $i$-te Rasterposition mit $1<=i<=N$ die folgenden Schritte i1) bis i4) durchgeführt. Auf der anderen Seite dauert die Bestrahlung einer Rasterposition typischerweise nur einige Millisekunden. Es ist also ersichtlich, dass aufgrund der Vieldimensionalität der Daten eine große Rechenleistung erforderlich ist.

i1) Die Recheneinrichtung ermittelt in Echtzeit während des Bestrahlungsdurchlaufs unter Verwendung der Bewegungsdaten und unter Verwendung der im Vorfeld, d.h. offline erstellten Datenbasis, die alle gegenseitigen Dosisbeiträge beinhaltet, diejenige Dosis, welche die $i$-te Rasterposition bei der Bestrahlung der vorherigen Rasterpositionen $(1<=k<i)$ bereits erhalten hat.

i2) Nachfolgend zu i1) und zwar unmittelbar vor der Bestrahlung der $i$-ten Rasterposition berechnet die Recheneinrichtung in Abhängigkeit von der ermittelten Dosis, welche die $i$-te Rasterposition bei der Bestrahlung der vorherigen Rasterpositionen $(1<=k<i)$ bereits erhalten hat, einen Kompensationswert für die $i$-te Rasterposition,

i3) Nachfolgend zu i2) und noch immer unmittelbar vor der Bestrahlung der $i$-ten Rasterposition wird in Abhängigkeit von dem Kompensationswert für die $i$-te Rasterposition und von dem im Bestrahlungsplan festgelegten Referenzdatentupel mit der nominellen Teilchenfluenz $Fi_{nom}$ für die $i$-te Rasterposition ein verändertes Datentupel mit kompensierter Teilchenfluenz $Fi_{komp}$ für die $i$-te Rasterposition berechnet.

i4) Nachfolgend wird die $i$-te Rasterposition mit der in Echtzeit berechneten kompensierten Teilchenfluenz $Fi_{komp}$ bestrahlt.

Mit dem erfindungsgemäßen Verfahren kann in vorteilhafter Weise trotz Bewegung des Zielvolumens eine ausreichende Dosisabdeckung erreicht werden und lokale Abweichungen von den Sollwerten reduziert werden, so dass der Einfluss der Bewegung auf die deponierte Ist-Dosisverteilung reduziert werden kann.

Insbesondere wird die Berechnung des Kompensationswertes für die $i$-te Rasterposition während der Bestrahlung der $i$-1-ten Rasterposition durchgeführt.

Vorzugsweise werden während der Bestrahlung die Dosisänderungen über alle $k<i$ aufsummiert, welche die $i$-te Rasterposition bei der Bestrahlung aller vorher bestrahlter Rasterpositionen $k$ erhalten hat und aus der Summe der Dosisänderungen (Differenz zwischen Ist- und Referenzdosis) wird der Kompensationswert für die Bestrahlung der $i$-ten Rasterposition während der Bestrahlung der $i$-1-ten Rasterposition berechnet.

Gemäß einer bevorzugten Ausführungsform der Erfindung berechnet die Recheneinrichtung den Kompensationswert für die $i$-te Rasterposition als einen relativen Korrekturfaktor, in dem die aufsummierten Dosisänderungen, die die $i$-te Rasterposition bei der Bestrahlung aller vorherigen $k$-ten Rasterpositionen erhalten hat, mit einer spezifischen Referenzdosis für die $i$-te Rasterposition normiert wird, so dass der Kompensationswert ein dimensionsloser Korrekturfaktor ist. Dieser Kompensationsfaktor wird vor der Bestrahlung der $i$-ten Rasterposition in einem Subsystem des Therapiekontrollsystems, nämlich dem Subsystem zur Kontrolle der Teilchenfluenz, das sogenannte SKT, gespeichert und anschließend ebenfalls noch vor der Bestrahlung der $i$-ten Rasterposition als der Kompensationswert für die $i$-te Rasterposition aus dem Speicher des Subsystems zur Kontrolle der Teilchenfluenz geladen und in Echtzeit mit der im Bestrahlungsplan festgelegten nominellen Teilchenfluenz für die $i$-te Rasterposition multipliziert, um die kompensierte Teilchenfluenz ($Fi_{komp}$) für die $i$-te Rasterposition zu berechnen, mit der dann bestrahlt wird. In vorteilhafter Weise wird der Kompensationswert dadurch unabhängig von täglich veränderlichen Kalibrationsfaktoren, die sich beispielsweise durch die Dosimetrie ergeben.

Vorzugsweise steuert das Subsystem zur Kontrolle der Teilchenfluenz SKT die applizierte Teilchenfluenz für alle Rasterpositionen und die Recheneinrichtung speichert den Kompensationswert für die i-te Rasterposition während der Bestrahlung der $i$-1-ten Rasterposition in dem Speicher des Subsystems zur Kontrolle der Teilchenfluenz SKT, so dass unmittelbar vor der Bestrahlung der $i$-ten Rasterposition der Kompensationswert für die $i$-te Rasterposition von dem Subsystem zur Kontrolle der Teilchenfluenz aus dem Speicher geladen werden kann. Das Subsystem SKT wendet dann in Echtzeit den Kompensationswert auf die im Bestrahlungsplan festgelegte nominelle Teilchenfluenz für die $i$-te Rasterposition an, um nachfolgend die $i$-te Rasterposition mit der kompensierten Teilchenfluenz für die $i$-te Rasterposition zu bestrahlen.

Wenn der Kompensationswert für die $i$-te Rasterposition zum geplanten Beginn der Bestrahlung der $i$-ten Rasterposition noch nicht in dem Speicher des Subsystems gespeichert ist, könnte die Strahlintensität verändert oder eine Strahlpause eingefügt werden, zumindest solange bis der Kompensationswert für die $i$-te Rasterposition gespeichert ist, um zu verhindern dass die $i$-te Rasterposition mit einer falschen Teilchenfluenz bestrahlt wird.

Um die Rechenleistung optimal auszunutzen und sicherzustellen, dass der Kompensationswert so früh wie möglich zur Verfügung steht, werden während der Bestrahlung der $i$-ten Rasterposition vorzugsweise nicht gleichzeitig die Dosisänderungen zu allen folgenden Rasterpositionen o, $i<o<=N$, sondern zunächst nur die Dosisänderung und der Kompensationswert für die $i$+1-te Rasterposition berechnet. Die Berechnung der Dosisänderungen und der Kompensationswerte für die folgenden Rasterpositionen $i$+1<$o$<=$N$, wird erst dann durchgeführt, wenn der Kompensationswert für die $i$-te Rasterposition in dem Speicher des Subsystems gespeichert ist.

Alternativ kann es sinnvoll sein, während der Bestrahlung der $i$-ten Rasterposition die Dosisänderungen für die Rasterpositionen der Isoenergieschicht, in der die $i$-te Rasterposition liegt, zu berechnen und die Dosisänderungen für die Rasterpositionen der folgenden Isoenergieschichten in einer Strahlpause (sogenannte Spillpause) zwischen der Bestrahlung der Isoenergieschichten zu berechnen.

Es kann nun vorkommen, dass die $i$-te Rasterposition bei der Bestrahlung der vorherigen $k$-ten Rasterpositionen bereits eine Dosisänderung erhalten hat, welche bereits größer oder gleich der im Bestrahlungsplan festgelegten Referenzdosis für die $i$-te Rasterposition ist. In diesem Fall wird die Bestrahlung der $i$-ten Rasterposition entweder übersprungen oder mit einer im Vorfeld der Bestrahlung festgelegten Mindestdosis bestrahlt. Letzteres kann vorteilhaft sein, um weiterhin die Strahllage zu kontrollieren. Hierzu sollte die Mindestdosis vorzugsweise größer oder gleich 1% der Referenzdosis, aber vorzugsweise kleiner oder gleich 30% der Referenzdosis sein.

Weiter vorteilhaft ist es, die Intensität des Strahls während der Bestrahlung rasterpositionsabhängig an die zu applizierende Teilchenfluenz der jeweiligen Rasterposition anzupassen. Dadurch kann die Bestrahlungsdauer der Rasterpositionen aneinander angeglichen werden, so dass die Zeit die zur Berechnung des Kompensationswertes zur Verfügung steht möglichst nivelliert wird und/oder an das Minimum herangeführt wird, um eine möglichst schnelle und damit fehlerarme und schonende Bestrahlung zu erreichen. Typischerweise wird die Bestrahlung einer Isoenergieschicht mit einem Strahlpaket (sogenannter Spill) durchgeführt und die nächste Isoenergieschicht erst mit dem nächsten Strahlpaket (Spill). Diesbezüglich ist es vorteilhaft, einen Qualitätsindex Q zu definieren, anhand dessen automatisch der Start des nächsten Strahlpakets (Spills) mit einer optimalen Bewegungsphase synchronisiert wird. Hierzu berechnet die Recheneinrichtung die rasterpositionsabhängige Verteilung der Teilchenfluenzen für die folgenden Rasterpositionen des nächsten Strahlpakets (Spills) für verschiedene Bewegungsphasen und bewertet mittels des Qualitätsindexes automatisch die Synchronisierung, z.B. anhand der folgenden Bewertungskriterien.

In den Qualitätsindex Q fließt vorzugsweise mindestens eines der folgenden Bewertungskriterien oder eine ggf. gewichtete Kombination dieser Bewertungskriterien ein:

i) die Ähnlichkeit der kompensierten Teilchenfluenzen der Rasterpositionen. Das hat den Vorteil, dass die Bestrahlungsdauer der Rasterpunkte möglichst ähnlich wird, so dass dies auch für die zur Verfügung stehende Rechenzeit gilt.

ii) Die Abweichungen der kompensierten Teilchenfluenzen von den Teilchenfluenzen der Referenzdosen sind möglichst klein. Hierdurch können ggf. Interferenzeffekte verringert werden.

iii) Eventuelle Überdosierungen der nachfolgenden Rasterpositionen werden reduziert. Hierdurch wird eine Dosierung erreicht die für jede Rasterposition möglichst nahe am Referenzwert liegt.

iv) Die Zeit bis zum voraussichtlichen Erreichen der zugehörigen Bewegungsphase. Typischerweise dauert im Mittel die Bestrahlung einer Rasterposition im Millisekunden-Bereich, einer Schicht im Sekundenbereich und einer Fraktion im Minuten-Bereich. Daher können die Bewegungsphasen erheblich länger als die Zeit für die Bestrahlung einer Rasterposition dauern. Wenn sich daher der Start der Bestrahlung mit dem nächsten Strahlpaket (Spill) zu sehr verzögern würde, kann dies ein weiterer Gewichtungsfaktor für den Qualitätsindex sein.

Die vorliegende Erfindung kann mit der aktiven Nachführung des Strahls an die Bewegung (Tracking) kombiniert werden, sie ist aber auch eine weniger aufwändige Alternative. Eine bevorzugte Anwendung der Erfindung ist es, zwar lateral aktive Strahlnachführung (Tracking) zu betreiben, aber nicht longitudinal. D.h. mit der durch die Messung eines Bewegungssurrogats ermittelten Bewegungsphase und der in der Bestrahlungsplanung ermittelten Parameter werden die rasterpositionsabhängigen Kompensationswerte bestimmt. Aktiv nachgeführt wird aber nur die laterale Strahlposition was relativ einfach über Scanner-Magnetpaare erfolgt. Die Strahlenergie hingegen wird nicht aktiv nachgeführt, sondern in der longitudinalen Dimension kompensiert das erfindungsgemäße Verfahren das Fehlen des aktiven Nachführens.

Weiter verbessern kann man die Wirkungsweise der Erfindung dadurch, dass sie mit einer Bestrahlung mit mehreren Bestrahlungsdurchläufen kombiniert wird, ähnlich der Mehrfachbestrahlung, bei welcher die Rasterpositionen innerhalb einer Fraktion mehrfach nacheinander abgetastet werden (Rescanning). Es wird also in jedem Bestrahlungsdurchlauf mit jeweils einem Bruchteil der Referenzdosis bestrahlt. Bei diesem Verfahren können bei der Berechnung des Kompensationsfaktors für die $i$-te Rasterposition in einem Bestrahlungsdurchlauf, die Dosisänderungen berücksichtigt werden, welche die $i$-te Rasterposition in den vorherigen Bestrahlungsdurchläufen erhalten hat und welche die $i$-te Rasterposition während der Bestrahlung der vorherigen Rasterpositionen in dem aktuellen Bestrahlungsdurchlauf erhalten hat. Hiermit kann die Ist-Dosisverteilung noch besser an die Soll-Dosisverteilung angeglichen werden. Z.B. kann die Anzahl der Bestrahlungsdurchläufe im Vorfeld anhand von Kollektivdaten und den Bewegungsparametern optimiert werden, patientenspezifisch sein, oder während der Bestrahlung ermittelt werden, indem beispielsweise eine Fehldosistoleranz von 0,1% oder 1% oder 10% der pro Fraktion geplanten Dosis akzeptiert wird. Sobald diese Toleranz unterschritten wird, wird die Bestrahlung beendet. Ausschlaggebend für die Beendigung können auch technische Randbedingungen wir die Bestrahlbarkeit eines inhomogenen Rasters (mehrere vereinzelte Rasterpunkte pro Isoenergieschicht) sein.

Im Folgenden wird die Erfindung anhand von Ausführungsbeispielen und unter Bezugnahme auf die Figuren näher erläutert, wobei gleiche und ähnliche Elemente teilweise mit gleichen Bezugzeichen versehen sind und die Merkmale der verschiedenen Ausführungsbeispiele miteinander kombiniert werden können.

Kurzbeschreibung der Figuren

Es zeigen:

Fig. 1    eine Darstellung der relativen Dosis als Funktion der Eindringtiefe für verschiedene Strahlsorten und Strahlenergien,

Fig. 2    eine schematische Darstellung einer Bestrahlungsvorrichtung mit Beschleunigeranlage mit drei Bestrahlungsplätzen,

Fig. 3    eine schematische Darstellung einer Anordnung zur Ermittelung der Positionsadaptionsparameter,

Fig. 4    eine schematische Darstellung einer Einrichtung zur dreidimensionalen aktiven Strahlnachführung (Tracking),

Fig. 5    eine Darstellung eines zu bestrahlenden Patienten am Bestrahlungsplatz,

Fig. 6a   eine schematische Darstellung der Bestrahlung einer Strahlposition mit einem nadelfeinen Ionenstrahl,

Fig. 6b   eine schematische Darstellung der Bestrahlung einer Strahlposition mit einem nadelfeinen Ionenstrahl unter Bewegungseinfluss,

Fig. 6c   eine schematische Darstellung der Bestrahlung einer Strahlposition mit einem nadelfeinen Ionenstrahl unter Bewegungseinfluss nach aktiver Strahlnachführung,

Fig. 7    eine schematische Darstellung der Bestrahlungsvorrichtung mit Steuerungselementen,

Fig. 8    eine Darstellung der Berechnung des Kompensationswertes,

Fig. 9    ein Ablaufplan für die Berechnung des Kompensationswertes.

Detaillierte Beschreibung der Erfindung

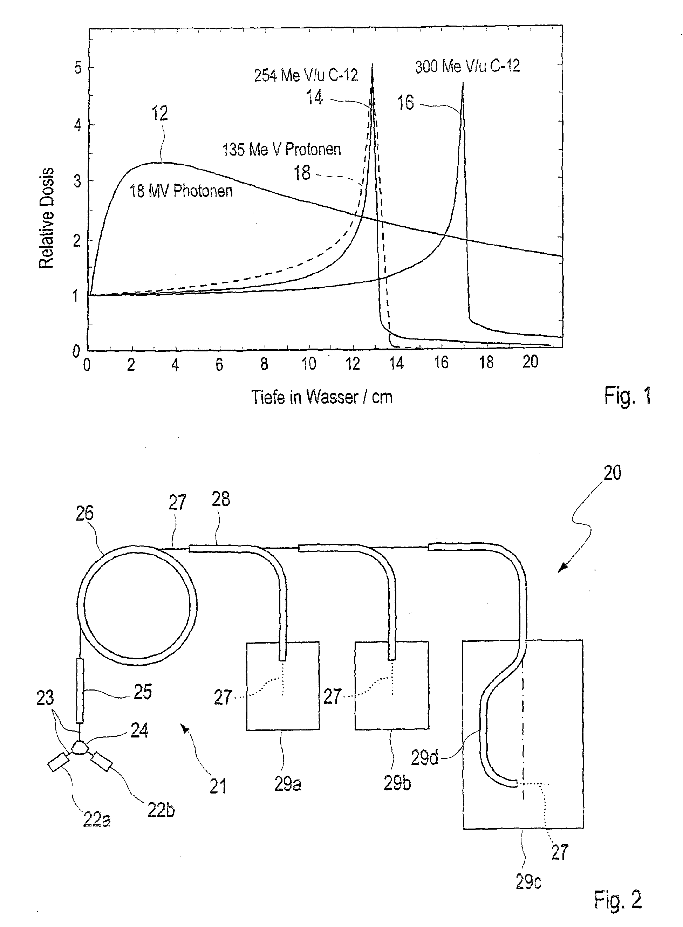

Bezug nehmend auf Fig. 1 ist die Tiefendosisverteilung für verschiedene Strahlarten dargestellt. Im Unterschied zu Photonen (Kurve 12), die nach einem Aufbaueffekt einen exponentiellen Abfall der Dosis mit der Tiefe aufweisen, zeigen Ionen am Ende der Strahlreichweite ein ausgeprägtes Dosismaximum, welches als Bragg-Peak oder Bragg-Maximum bezeichnet wird. Dieses Maximum kann durch Energievariation in der Tiefe verschoben werden. Es ist erkennbar, dass das Bragg-Maximum für Kohlenstoffionen (Kurven 14 und 16) schärfer ist als für Protonen (Kurve 18).

Fig. 2 zeigt einen schematischen Überblick über den Aufbau einer exemplarischen Bestrahlungsvorrichtung 20 zum Bestrahlen eines Zielvolumens in einem Körper. Die Bestrahlungsvorrichtung umfasst eine Beschleunigeranlage 21, in diesem Beispiel mit zwei Partikel- oder Ionenquellen 22a, 22b, welche unterschiedliche Ionensorten erzeugen und deren niederenergetischer intermediärer Ionenstrahl 23 über einen Schaltmagneten 24 in einen Linear-Vorbeschleuniger 25 injiziert wird. Der intermediäre Ionenstrahl 23 wird mit dem Linear-Vorbeschleuniger 25 vorbeschleunigt und in einen Kreisbeschleuniger 26, z.B. ein Synchrotron injiziert. Alternativ kann auch ein Zyklotron, ein sogenannter "Dielectric-Wall-Beschleuniger" oder ein Laser-basierter Beschleuniger verwendet werden. Manche Beschleuniger sind sogar direkt im Bestrahlungsraum, z.B. auf einer Gantry installierbar, so dass keine separate Strahlführung notwendig ist.

Der Kreisbeschleuniger 26 beschleunigt die Ionen schließlich auf eine Sollenergie, welche der Behandlungsenergie oder etwas darüber entspricht. Der Ionenstrahl oder Behandlungsstrahl 27 wird anschließend aus dem Kreisbeschleuniger 26 extrahiert und über Strahlführungseinrichtungen 28 in Bestrahlungskammern 29a, 29b, 29c geleitet, in welchen jeweils ein Körper 30 mit dem Ionenstrahl 27 bestrahlt werden kann. Für eine Bestrahlung mit Kohlenstoffionen liegt die Teilchenenergie des Behandlungsstrahls 27 im Bereich von etwa 80 bis 500 MeV/u. In den beiden linken Bestrahlungskammern 29a, 29b wird das Zielvolumen 32 in dem Körper 30 im Wesentlichen in der Richtung bestrahlt, in der der Ionenstrahl 27 in die Bestrahlungskammer 29a, 29b eintritt und die rechte Bestrahlungskammer 29c weist eine sogenannte drehbare Gantry 29d auf, mit welcher das Zielvolumen 32 aus unterschiedlichen Richtungen bestrahlt werden kann (isozentrische Bestrahlung). Der Körper 30 ist z.B. ein Patient und das Zielvolumen 32 ein im Rahmen einer Tumorbehandlung zu bestrahlender Tumor. Der Körper kann allerdings auch ein Phantom zur Validierung einer Tumorbestrahlung sein, und das Zielvolumen z.B. ein Detektor oder andere zu bestrahlende Materie.

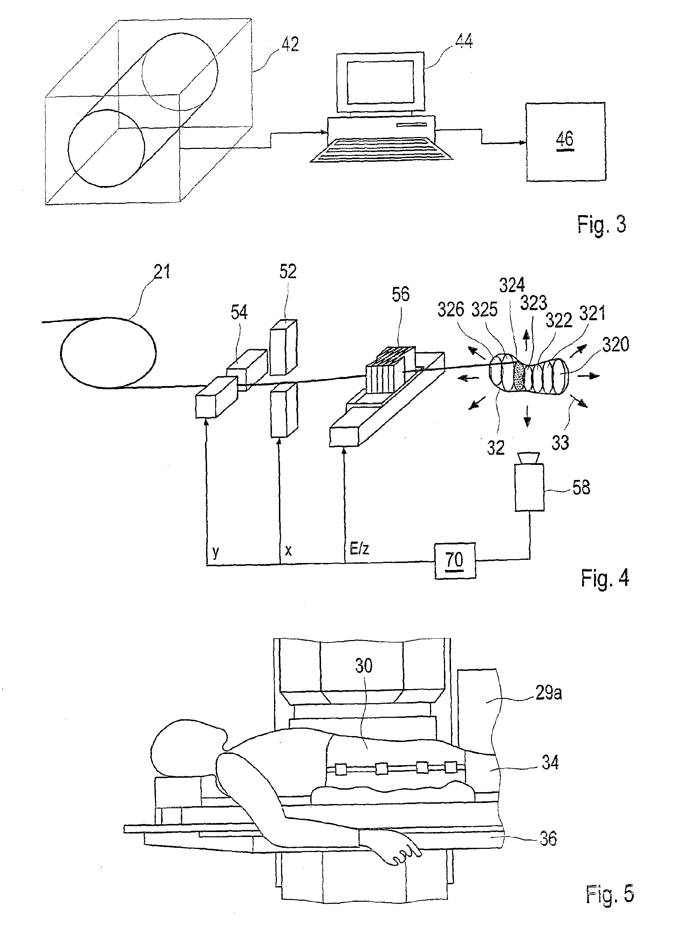

Fig. 3 zeigt einen zeitauflösenden und dreidimensional bildgebenden Tomographen 42, z.B. ein sogenanntes 4D-CT oder ein sogenanntes 4D-MRT mit Bewegungssensoren und Recheneinrichtung. Der Tomograph nimmt die 3-dimensionale Bewegung des Zielvolumens 32, z.B. des Tumors unter dem Einfluss der Bewegung des Körpers 30, z.B. der Atmung des Patienten, auf und unterteilt die Bewegungsdaten in M Bewegungsphasen. Mit dem Computer 44 wird dann auf Basis der Sollgesamtdosisdeposition im Vorfeld der Bestrahlung ein statischer Bestrahlungsplan 46 erstellt, welcher die Positions- und nominellen Teilchenfluenzdaten für jede zu bestrahlende Rasterposition enthält.

Der Computer 44 erstellt ferner eine Positionsadaptionstabelle mit dreidimensionalen Positionsadaptionsparametern ($\Delta x_i$, $\Delta y_i$, $\Delta E_i$) jeweils für jede Bewegungsphase $m$ ($1<=m<=M$), so dass anhand dieser Positionsadaptionstabelle und unter Kenntnis der Bewegung des Körpers die tatsächliche Position jeder einzelnen Rasterposition bzw. die Abweichung der Ist-Position von einer Referenzposition in einer Referenzbewegungsphase *m=ref* berechnet werden kann. Hierbei steht $\Delta x_i$ für die Abweichung der lateralen *x*-Position der Rasterposition *i* in der Bewegungsphase *m* von der *x*-Position der Rasterposition *i* in der Referenzphase *m*=ref. Entsprechend definiert $\Delta y_i$ die Abweichung in der anderen lateralen Richtung, nämlich *y*-Richtung. $\Delta E_i$ definiert die Abweichung der Ionenenergie, um das Bragg-Maximum von der Referenzposition der Rasterposition *i* in deren Istposition zu verschieben, also den longitudinalen Positionsadaptionsparameter.

Fig. 4 zeigt eine vereinfachte schematische Darstellung einer Bestrahlung mit aktiver Bewegungskompensation. Die Beschleunigeranlage 21 stellt den Behandlungsstrahl 27 zur Verfügung, welcher mit zwei Magnetscannerpaaren 52, 54 lateral über das Zielvolumen 32 gerastert wird. Das Zielvolumen ist unterteilt in eine Mehrzahl von Isoenergieschichten, hier 318-324, welche nacheinander abgerastert werden. In der Regel ist es sinnvoll von den distalen in Richtung der proximalen Isoenergieschichten abzurastern, d.h. mit der höchsten Energie zu beginnen. In der Fig. 4 wird in einer Momentaufnahme die Isoenergieschicht 322 lateral abgetastet. Bewegt sich das Zielvolumen 32 wie durch die Pfeile 33 symbolisiert ist, wird die Strahlposition bei Kenntnis der Bewegung der aktuell bestrahlten Rasterposition i lateral mittels der Scannermagnete 52, 54 und longitudinal mittels eines Doppelkeilsystems 56 aktiv der Bewegung des Zielvolumens 32 nachgeführt, um trotz Bewegung des Zielvolumens 32 die beabsichtigte Rasterposition *i* zu treffen. Die Istposition der gerade bestrahlten Rasterposition *i* wird bestimmt mittels einer Bewegungserfassungseinrichtung 58, die die Bewegung des Körpers 30 erfasst und mittels der im Therapiekontrollsystem 70 gespeicherten Positionsadaptionstabelle.

Fig. 5 zeigt einen Patienten 34 auf einer Patientenliege 36 z.B. in der Bestrahlungskammer 29a. Anstatt des lebenden Patienten 34 kann auch ein anderer Körper 30, z.B. ein Phantom bestrahlt werden.

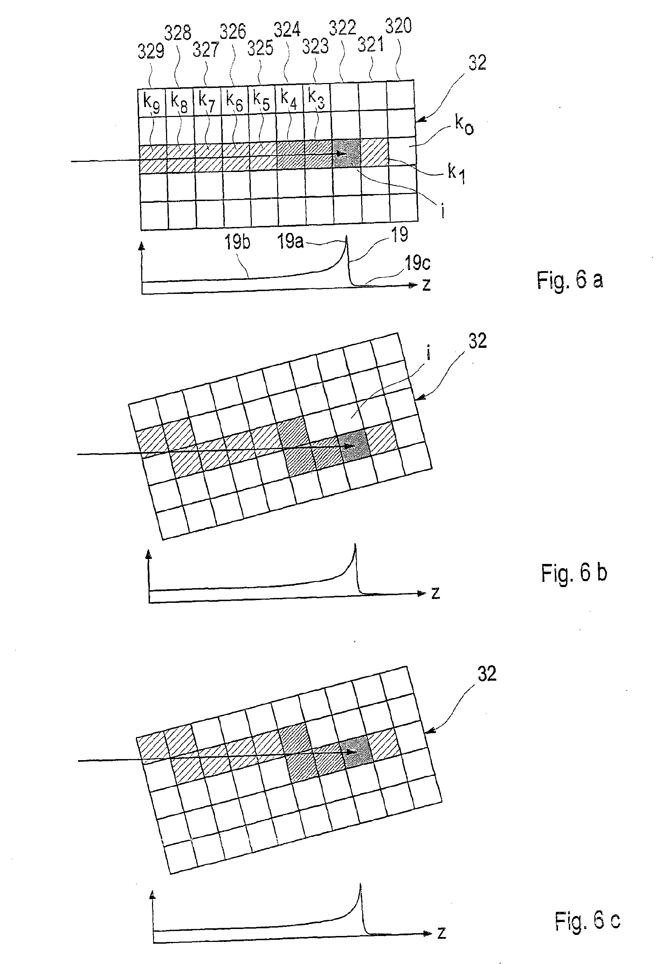

Bezug nehmend auf die Fig. 6a soll momentan die Rasterposition *i* bestrahlt werden. Das bedeutet, dass der Strahl lateral (*x*, *y*) auf die Rasterposition *i* gerichtet wird und die Energie (*E*) der Partikel so eingestellt wird, dass das Bragg-Maximum in der Isoenergieschicht 322 liegt, in der die momentane Rasterposition *i* liegt. Insoweit zeigt die Fig. 6a die Bestrahlung eines ruhenden Zielvolumens 32.

Anhand der Dosisdeposition 19 als Funktion der Eindringtiefe *z* ist ersichtlich, dass trotz des scharfen Bragg-Maximums 19a auch andere Rasterpositionen als die Rasterposition *i* eine Dosisdeposition erhalten, insbesondere diejenigen Rasterpositionen im Plateaubereich 19b strahlaufwärts des Bragg-Maximums 19a im Strahlweg, allerdings auch in etwas geringerem Maße die Positionen auf der Strahlachse strahlabwärts des Bragg-Maximums 19a der Rasterposition *i*, also im Bereich 19c hinter dem Bragg-Maximum 19a. Diese Dosisdeposition in den anderen Rasterpositionen $k_0$, $k_1$ und $k_3$ bis $k_9$ bei ruhendem Zielvolumen 32 sind in dem ("statischen") Referenz-Bestrahlungsplan 46 bereits berücksichtigt. Der im Vorfeld der Bestrahlung erstellte Bestrahlungsplan 46 enthält nämlich eine Tabelle in der festgelegt ist, welche Teilchenanzahl für jede Rasterposition vorgesehen ist, um für jede Rasterposition eine vordefinierte Dosisdeposition zu erzielen. In diesem Bestrahlungsplan 46 ist bereits die Dosisdeposition berücksichtigt, welche eine Rasterposition *i* bei der Bestrahlung aller anderen Rasterpositionen k erhält, z.B. durch das Plateau 19b vor dem Bragg-Maximum 19a, aber unter der Voraussetzung eines ruhenden Zielvolumens 32.

Bewegt sich nun das Zielvolumen, z.B. wie in Fig. 6b dargestellt ist, so dass sich die eigentlich zu bestrahlende Rasterposition *i* nach oben verschiebt und sich das Zielvolumen zusätzlich verkippt, entstehen zwei nachteilige Effekte, nämlich erstens trifft das Bragg-Maximum nicht mehr die eigentlich zu bestrahlende Rasterposition *i* und zweitens verändert sich der Strahlweg durch das Zielvolumen 32 z.B. durch die Verkippung des Zielvolumens. Ferner kann sich das Zielvolumen 32 auch deformieren, was ebenfalls den Strahlweg verändert.

Mittels der aktiven Nachführung der Strahlposition kann dafür gesorgt werden, dass das Bragg-Maximum 19a wieder in die Rasterpositionen *i* geschoben wird, wie in Fig. 6c dargestellt ist. Dieses Verfahren, den Strahl 27 aktiv nachzuführen ist grundsätzlich bekannt und wird in der Fachwelt als "Tracking" bezeichnet. Wie in Fig. 6c aber noch weiter zu erkennen ist, nimmt der Strahl trotzdem noch einen anderen Weg durch das Zielvolumen als in der Bestrahlungsplanung unter der Annahme eines ruhenden Zielvolumens gemäß Fig. 6a, so dass die Dosisdeposition z.B. des Plateaubereichs 19b in anderen Rasterpositionen k stattfindet, als bei einem ruhenden Zielvolumen, obwohl der Strahl aktiv der Bewegung des Zielvolumens 32 nachgeführt wird. Diese Fehldeposition lässt sich durch aktive Nachführung des Strahls 27 alleine nicht vermeiden und ist in dem statischen Bestrahlungsplan 46 nicht berücksichtigt. Hier setzt die Erfindung an.

Im Folgenden wird die Erfindung anhand von Bezugnahmen auf einzelne Rasterpositionen beschrieben, welche mit Variablen wie *i, k* oder anderen Platzhaltern bezeichnet sind, um diese untereinander in Beziehung zu setzen. Es ist ersichtlich, dass sich diese Platzhalter als Variablen auf alle Rasterpositionen, also z.B. $1<=i<=N$ beziehen.

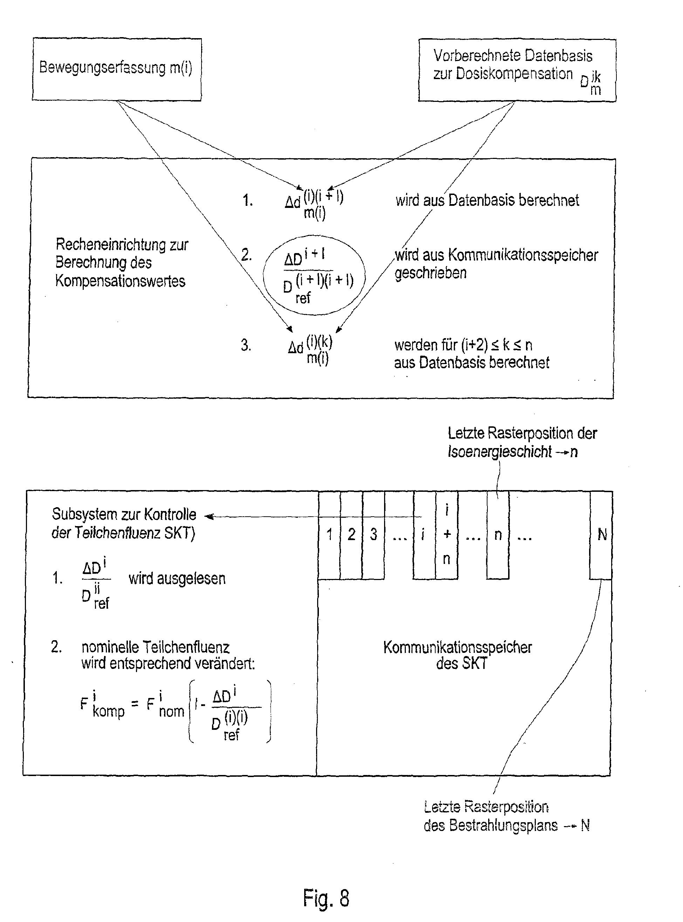

Der Bestrahlungsplan besteht aus *N* Rasterpositionen und enthält *M* Bewegungsphasen. Bezug nehmend auf die in Fig. 8 gezeigte Darstellung wird die Dosis, die während der Bestrahlung von Rasterposition *i* in Bewegungsphase *m* an Rasterposition *k* deponiert wird, im Vorfeld der Bestrahlung vorberechnet und mit $D_m^{ik}$ bezeichnet. Hierbei wird angenommen, dass an Rasterposition *i* eine Dosis gemäß der Bestrahlungsplanung deponiert wird. Für jede Rasterposition $i$ werden diese Werte für alle noch zu bestrahlenden Rasterpositionen $k$ und für jeweils alle möglichen Bewegungsphasen $1 \leq m \leq M$ vor der Bestrahlung berechnet. Wie in Bezug auf Abb. 4 aufgeführt, wird in der Regel von distal nach proximal bestrahlt, damit sich proximal ergebende Änderungen bei der Bestrahlung von distalen Rasterpositionen bei der später erfolgenden Bestrahlung der proximalen Rasterpositionen korrigiert werden können.

Während der Bestrahlung einer Rasterposition $i$ werden die hierbei verursachten Dosisänderungen $\Delta d^{ik}_{m(i)}$ aller später bestrahlter Rasterpositionen $k$, bei diesen kann die Dosierung noch aktiv verändert werden, nun erfindungsgemäß in Abhängigkeit der Bewegung jeder einzelnen Rasterposition, nach folgender Formel berechnet:

$$\Delta d^{ik}_{m(i)} = \left( D^{ik}_{m(i)} \left( 1 - \frac{\Delta D^i}{D^{ii}_{ref}} \right) - D^{ik}_{ref} \right), \qquad (1)$$

wobei $m(i)$ die gemessene Bewegungsphase während der Bestrahlung der Rasterposition $i$ und $\Delta D^i$ die im bisherigen Bestrahlungsverlauf akkumulierte Dosisänderung für Rasterposition $i$ bezeichnet, die in Echtzeit während der Bestrahlung durch Summation der Einzelbeiträge aller bisher bestrahlter Rasterpositionen zu Rasterposition $i$ berechnet wird:

$$\Delta D^i = \sum_{l=0}^{i-1} \Delta d^{li}_{m(l)} . \qquad (2)$$

$\Delta D^i$ betrifft somit die Dosisänderung an der Rasterposition i, die als Kompensationswert verwendet werden kann.

Im Term $\dfrac{\Delta D^i}{D^{ii}_{ref}}$ in Formel (1) wird die akkumulierte Dosisänderung auf die Dosis $D^{ii}_{ref}$, die die Rasterposition $i$ laut Bestrahlungsplanung bei der Bestrahlung der Rasterposition $i$ erhalten soll, normiert. Dabei steht der Index "ref" für die Referenzphase $m = ref$. Die tatsächliche momentane Position jeder Rasterposition in jeder Bewegungsphase $m$ ist aufgrund der Positionsadaptionstabelle, welche vor der Bestrahlung (offline) bei der Bestrahlungsplanung festgelegt wurde berechenbar.

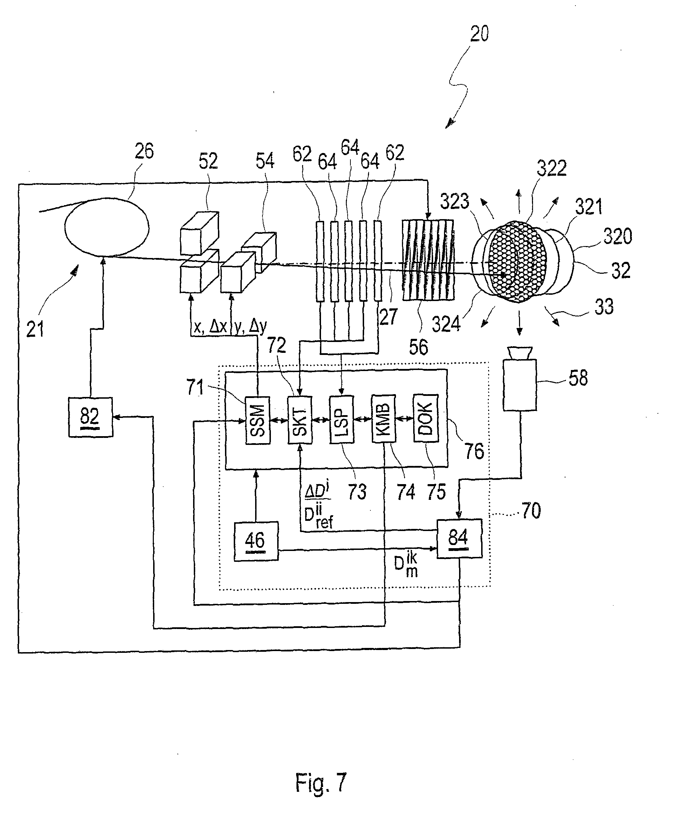

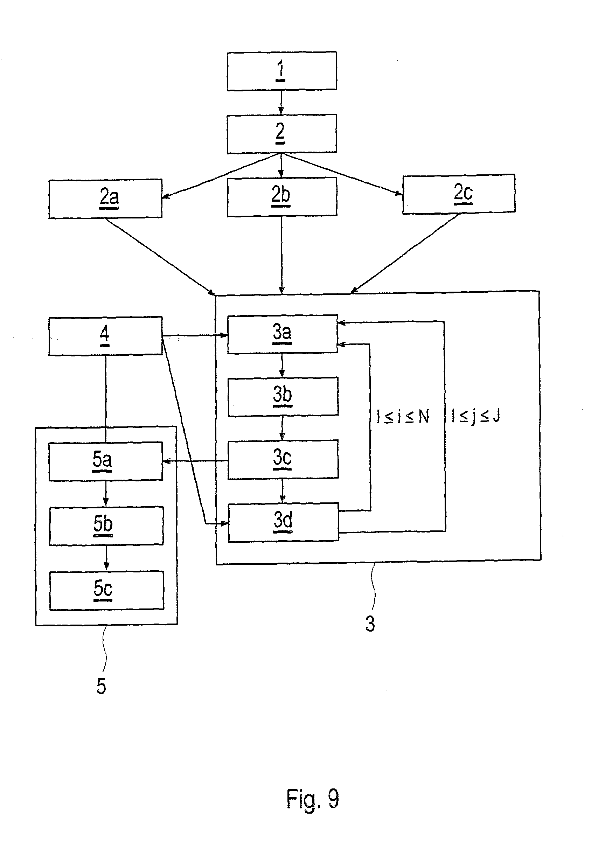

Während der Bestrahlung der Rasterposition $i$ wird die normierte, und damit relative, Dosisänderung der nächsten Rasterposition $\dfrac{\Delta D^{i+1}}{D^{(i+1)(i+1)}_{ref}}$ an das Subsystem zur Kontrolle der Teilchenfluenz (SKT) 72 (vgl. Fig. 7 und 9) weitergegeben. Im Bestrahlungs-Kontrollsystem wird hierfür der Kommunikationsspeicher des SKT 72 genutzt, der während einer laufenden Bestrahlung sowohl vom SKT 72 als auch von anderen Subsystemen 71, 73, 74, 75 zugänglich ist. An anderen Beschleunigeranlagen können auch andere Kommunikationsprotokolle und -geräte genutzt werden, die zur Echtzeitkommunikation in der Lage sind.

Durch die Nutzung der dimensionslosen relativen Dosisänderung $\dfrac{\Delta D^i}{D^{ii}_{ref}}$ als Kompensationswert kann die Dosisänderung vom SKT 72 in vorteilhafter Weise ohne Umrechnung von Dosiseinheiten in Maschinenparameter angewendet werden. Zusätzlich ist diese relative Größe unabhängig von täglich veränderlichen Kalibrationsfaktoren der zur Dosisüberwachung verwendeten Ionisationskammern 64.

Die nominelle Teilchenfluenz aus der Bestrahlungsplanung $F^i_{nom}$ wird mit dem Kompensations faktor

$$\left(1 - \frac{\Delta D^i}{D^{ii}_{ref}}\right)$$ multipliziert d.h. für die nächste Rasterposition $i$+1 wird dann die um die akkumulierte Dosisänderung

kompensierte Teilchenfluenz $F^{i+1}_{komp} = F^{i+1}_{nom}\left(1 - \frac{\Delta D^{i+1}}{D^{(i+1)(i+1)}_{ref}}\right)$ angewendet.

Die kompensierte Teilchenfluenz spiegelt also genau den Dosisbeitrag wieder, der noch benötigt wird, damit die Rasterposition $i$+1 zumindest die in der Bestrahlungsplanung vorgesehene Referenzdosis erhält und zwar trotz Bewegung und trotz Änderung des Strahlweges. Entsprechend reflektiert der Kompensationsfaktor in Formel (1), dass die Bestrahlung der Rasterposition $i$ nicht mit der Referenzdosis sondern mit einer um den Kompensationsfaktor

$$\left(1 - \frac{\Delta D^i}{D^{ii}_{ref}}\right)$$ veränderten Dosis erfolgt.

Bezug nehmend auf den in Fig. 9 dargestellten Ablaufplan wird also wie folgt vorgegangen:

Position 1: Es wird eine zeitaufgelöste Tomographie (sogenannte 4D Diagnostik, z.B. 4D-CT) durchgeführt oder es werden zeitaufgelöste Tomographiedaten beispielsweise auf Basis eines 3DCTs und patientenspezifischen- oder Populations-Bewegungsdaten berechnet.

Position 2: Es wird im Vorfeld der Bestrahlung die Bestrahlungsplanung unter Berücksichtigung der Bewegungsphasen $m$ (sogenannte 4D Bestrahlungsplanung) auf Basis der Daten der zeitaufgelösten Tomographie wie folgt durchgeführt:

Position 2a: Es werden Datentupel $(x_i, y_i, E_i, T_i)$ für jede Rasterposition $1<=i<=N$ definiert, wobei $x_i$ und $y_i$ die lateralen Positionen, $E_i$ die Teilchenenergie und $T_i$ die zu deponierende Teilchenanzahl der $i$-ten Rasterposition sind. Es ist ersichtlich, dass Teilchenanzahl und Teilchenfluenz austauschbare Größen sind. Die Definition wird für einen oder $J$ Bestrahlungsdurchläufe mit jeweils $N$ Rasterpositionen im Vorfeld der Bestrahlung durchgeführt und das Ergebnis wird allgemein als Bestrahlungsplan bezeichnet.

Position 2b: Es werden für jede Rasterposition $i$ und für jede Bewegungsphase $m$ $(\Delta x_i, \Delta y_i, \Delta E_i)$ Positionsadaptionsparameter auf Basis der Daten der zeitaufgelösten Tomographie festgelegt, welche die Rückrechnung des tatsächlichen Ortes der Rasterpositionen bei Bewegung auf eine Referenzposition gestattet, wobei die Referenzposition in der Referenzphase $m = ref$ einem hypothetischen Ruhezustand entspricht.

Position 2c: Es werden Dosiskompensationsparameter $D^{ik}_m$ für alle Kombinationen $i, k = 1...N$ und alle Bewegungsphasen $m = 1...M$ definiert.

Hierbei werden folgende Schritte durchgeführt:

Ggf. Aufnahme eines 4D-MRT/4D-PET (Segmentierung, Staging);

Definition einer Referenzbewegungsphase, insbesondere bei Ausatmung;

Falls kein 4D-CT vorliegt, Aufnahme eines 4D-CTs;

Registrierung der M-1 Bewegungsphasen zur Referenzbewegungsphase durch (nicht-rigide) Transformationen (Optimierungsvorgang bei dem z.B. die normierte wechselseitige Information, sogenannte "normalized mutual information" minimiert wird). Die entstehenden M-1 Transformationen (und deren Inverse) können die 3D Bewegung des Tumors beschreiben;

Optimierung eines quasi-stationären Referenzbestrahlungsplans unter Verwendung der Referenzphase des 4D-CT (die 3-dimensional ist) und den segmentierten Volumina (Tumor und Risiko-Organ, sogenanntes "organ at risk", OAR) aus dem (4D)-MR oder dem Kontrastmittel-CT oder 4D-CT. Dies ist dem Fachmann grundsätzlich bekannt, z.B. aus Krämer et al. "Treatment planning for heavy-ion radiotherapy: physical beam model and dose optimization. Phys Med Biol 2000, 45:33299-3317, Krämer et al. "Treatment planning for heavy-ion radiotherapy: calculation and optimization of biologically effective dose. Phys Med Biol 2000, 45:3319-3330, Jäkel and Krämer, "Treatment planning for heavy ion irradiation", Phys. Med. 14 53-62;

Kombination von Referenzbestrahlungsplan, Transformationsparametern und 4D-CT Phasen, um für alle Rasterpunkte und alle Phasen Adaptionsparameter dx, dy, dE zu bestimmen;

Bestimmung der Dosisbeiträge von Rasterpunkt i zu Rasterpunkt k in Abhängigkeit von der Bewegungsphase m, nachfolgend $D_m^{ik}$ genannt;

Position 3: Während der Bestrahlung wird in Echtzeit für alle Rasterpositionen über alle 1<=i<=N und ggf. alle Bestrahlungsdurchläufe 1<=j<=J die Schritte 3a bis 3d in Kombination mit den Positionen 4 und 5 durchgeführt:

Position 3a: Berechnung von $\Delta d_{m(i)}^{(i)(i+1)}$ auf Basis der Dosiskompensationsparameter aus Position 2c und in Abhängigkeit der von der Bewegungserfassungseinrichtung erfassten Bewegungsdaten aus Position 4

Position 3b: Berechnung des relativen dimensionslosen Kompensationsfaktors $\dfrac{\Delta D^{i+1}}{D_{ref}^{(i+1)(i+1)}}$

Position 3c: Weitergabe des relativen dimensionslosen Kompensationsfaktors $\dfrac{\Delta D^{i+1}}{D_{ref}^{(i+1)(i+1)}}$ an das Subsystem zur Kontrolle der Teilchenfluenz SKT und Speicherung im Kommunikationsspeicher des SKT gemäß Position 5.

Position 3d: Berechnung der $\Delta d_{m(i)}^{ik}$ auf Basis des Dosiskompensationsparameters $D_m^{ik}$ aus Position 2c und in Abhängigkeit der von der Bewegungserfassungseinrichtung erfassten Bewegungsdaten aus Position 4 für alle noch zu bestrahlenden k>i+1.

Position 4: Erfassung der Bewegungsdaten mit der Bewegungserfassungseinrichtung.

Position 5: Steuerung der Teilchenfluenz mittels des Subsystems zur Kontrolle der Teilchenfluenz SKT 72.

Position 5a: Auslesen der Dosisänderung $\dfrac{\Delta D^{i+1}}{D_{ref}^{(i+1)(i+1)}}$ aus dem Speicher des SKT

Position 5b: Berechnung der kompensierten Teilchenfluenz $F_{komp}^{i+1}$ durch das SKT 72.

Position 5c: Bestrahlen der Rasterposition i+1. mit der kompensierten Teilchenfluenz $F_{komp}^{i+1}$.

Zusammenfassend wird also während der Bestrahlung in Echtzeit ein Kompensationswert berechnet und der Kompensationswert auf die nominelle Teilchenfluenz angewendet, um eine kompensierte Teilchenfluenz zu erhalten, mit welcher dann anstatt mit der im Bestrahlungsplan festgelegten nominellen Teilchenfluenz bestrahlt wird. Der Kompensationswert hängt von der tatsächlich unter Bewegung rasterpositionsspezifisch applizierten Dosis ab und ist somit rasterpostionsabhängig und bewegungsabhängig.

Die Erfindung löst auch die Kontrolle des zeitlichen Ablaufs der Dosiskompensation, welche relativ anspruchsvoll ist, und zwar vorzugsweise dadurch, dass während der Bestrahlung einer Rasterposition i dafür Sorge getragen wird, dass der Kompensationswert für die nachfolgende Rasterposition i+1 an das SKT weitergegeben wird, bevor das SKT den Kompensationswert anwendet.

Fig. 7 zeigt eine beispielhafte Implementierung der Erfindung. Der von der Beschleunigeranlage 21 erzeugte Ionenstrahl 27 wird auf das Zielvolumen 32 gerichtet, welches hier in Isoenergieschichten 320 bis 324 unterteilt ist und sich während der Bestrahlung intrafraktionell bewegt, wie durch die Pfeile 33 angedeutet ist. Der Strahl 27 wird lateral, d. h. in *x*- und *y*-Richtung mittels der Scannermagnetpaare 52, 54 über das Zielvolumen 32, in der Darstellung momentan über die Isoenergieschicht 322 gerastert.

Die tatsächliche Strahlposition wird über zumindest eine, in diesem Beispiel zwei Vieldrahtkammern 62 überwacht und die Positionsinformation an das Subsystem zur Überwachung der lateralen Strahlposition LSP 73 übermittelt. Falls die laterale Position des Strahls 27 nicht genau genug mit der Position gemäß dem Bestrahlungsplan 46 übereinstimmt, verursacht das LSP 73 einen Interlock.

Das Subsystem zur Steuerung der Scannermagnete SSM 71 steuert die Scannermagnete und damit die laterale Rasterposition in *x*- und *y*-Richtung.

Das Subsystem zur Kontrolle der Teilchenfluenz SKT 72 kontrolliert die tatsächlich deponierte Teilchenanzahl bzw. -fluenz und entscheidet mit Hilfe von Ionisationskammern 64, welche dreifach redundant ausgelegt sein können, aufgrund des Bestrahlungsplans 46 und anhand des Kompensationswertes, wann die aktuelle Rasterposition *i* genug Teilchen erhalten hat und auf die nächste Rasterposition *i*+1 weitergeschaltet werden kann. Der Steuerbefehl zum Weiterschalten wird von dem Modul SKT 72 unter anderem an das Modul SSM 71 kommuniziert, welches dann die Scannermagnete entsprechend ansteuert. Das Modul SKT 72 kommuniziert ebenfalls das Ende einer Isoenergieschicht.

Das Subsystem zur Kontrolle der Beschleunigeranlage KMB 74 kommuniziert mit der Steuereinrichtung 82 für die Beschleunigeranlage 21. Über diese Kommunikation fordert das Therapiekontrollsystem 70 z. B. das nächste Strahlpaket (Spill) mit den aktuell benötigten Parametern Teilchenenergie für die aktuelle Isoenergieschicht, Strahlfokus und Strahlintensität an. Das Therapiekontrollsystem 70 umfasst hier neben den Subsystemen 71 bis 74 noch ein weiteres Subsystem DOK 75 zur Dokumentation, sowie den Bestrahlungsplan 46 und eine Recheneinrichtung 84.

Während der Bestrahlung der Isoenergieschicht 322 erfasst die Bewegungserfassungseinrichtung 58 die Bewegung des Körpers 30 und übermittelt diese an die Recheneinrichtung 84 zur Berechnung des Kompensationswertes. Die Recheneinrichtung 84 berechnet nun in Echtzeit während der Bestrahlung der aktuellen Rasterposition *i* den bewegungsabhängigen Kompensationswert $\dfrac{\Delta D^{i+1}}{D_{ref}^{(i+1)(i+1)}}$ anhand der gemäß Fig. 3 erstellten Positionsadaptionstabelle und der ("stationären") Referenz-Dosiskompensationsparameter $D_m^{ik}$ .

Den Dosiskompensationsparameter $D_m^{ik}$ erhält die Recheneinrichtung 84 vom Bestrahlungsplan 46. die Recheneinrichtung 84 übergibt in Echtzeit je Rasterposition *i* den bewegungsabhängigen Kompensationswert $\dfrac{\Delta D^{i+1}}{D_{ref}^{(i+1)(i+1)}}$ an das Modul SKT 72. Das Modul SKT 72 berechnet anhand der nominellen Teilchenfluenz aus dem Bestrahlungsplan 46 und des Kompensationswertes aus der Recheneinrichtung 84 die kompensierte Teilchenfluenz in Echtzeit und kommuniziert mit dem Modul SSM 71 zur entsprechenden Steuerung des Strahls 27 auf die nächste Rasterposition *i*+1, wenn die kompensierte Teilchenfluenz deponiert wurde.

In dem vorliegenden Beispiel wird die erfindungsgemäße Dosiskompensation mit dreidimensionaler aktiver Strahlnachführung kombiniert, so dass die Recheneinrichtung 84 das Modul SSM 71 und dieses dann die Scannermagnete 52 und 54 steuert, in dem Δ*x*- und Δ*y*-Werte zum Nachführen des Strahls in lateraler Richtung bereitgestellt werden. Ferner steuert die Recheneinrichtung 84 das Doppelkeilsystem 56, über welches die longitudinale Strahlnachführung bewerkstelligt wird oder ein anderes System zum aktiven Nachführen der Energie.

Das Therapiekontrollsystem 70 umfasst in dem vorliegenden Beispiel ein VME-BUS-System 76, so dass die Module 71 bis 75 über den VME-BUS miteinander kommunizieren können.

1. Vorteilhafte Ausführungsformen

Die Erfindung kann wie folgt weitergebildet werden:

a) Während der Bestrahlung einer Rasterposition *i* werden nicht die Dosisänderungen zu allen folgenden Rasterpositionen *k*>i berechnet und dann der akkumulierte Kompensationswert für die nächste Rasterposition *i*+1 an das SKT 72 weitergegeben und dort gespeichert, sondern es wird zunächst nur der Dosisänderungsbeitrag für die nächste Rasterposition i+1 berechnet, und nachfolgend wird der akkumulierte Kompensationswert für diese Rasterposition *i*+1 weitergegeben. Erst danach werden die Beiträge zu allen übrigen Rasterpositionen *i*+2<=*k*<=*N* berechnet.

Hierdurch wird sichergestellt, dass der Kompensationswert für die Bestrahlung der nächsten Rasterposition, hier also *i*+1 so früh wie möglich im Kommunikationsspeicher des SKT 72 zur Verfügung steht.

b) Während der Bestrahlung einer Rasterposition werden nur die Dosisänderungen für Rasterpositionen derselben Isoenergieschicht berechnet. Dosisänderungs-Beiträge zu den übrigen Rasterpositionen in den anderen Isoenergieschichten des Bestrahlungsplans 46 werden erst während einer Strahlpause (Spillpause), die spätestens nach Ende der Isoenergieschicht stattfindet, berechnet. Dieses Verfahren eignet sich z.B. für Beschleunigeranlagen mit aktiver Energievariation. Ein Beispiel hierfür ist eine Beschleunigeranlage mit einem Synchrotron, bei welchem die Strahlenergie, welche die zu bestrahlende Isoenergieschicht definiert, aktiv am Synchrotron verändert wird. Dies geht mit der Erzeugung eines neuen Strahlpakets (Spill) einher, so dass zwischen der Bestrahlung der Isoenergieschichten ohnehin Strahlpausen vorhanden sind.

Andere Beschleunigeranlagen, z.B. mit einem Zyklotron, definieren de Strahlenergie mit Absorberplatten (passive Energievariation). Auch hier dauert das Ein- und/oder Herausfahren der Absorberplatten zur Änderung der Energie eine gewisse Zeit, was mit einer Strahlpause verbunden ist. Auch diese Strahlpause kann genutzt werden, um die Dosisänderungs-Beiträge zu den übrigen Rasterpositionen in den anderen Isoenergieschichten des Bestrahlungsplans zu berechnen.

Das Zurückstellen der Berechnung der Dosisänderungs-Beiträge der übrigen Rasterpositionen in den anderen Isoenergieschichten des Bestrahlungsplans verkürzt während der zeitkritischen Phase der sukzessiven Bestrahlung der Rasterpositionen innerhalb einer Isoenergieschicht die Rechenzeit und verringert somit die Wahrscheinlichkeit, dass die notwendigen Berechnungen länger in Anspruch nehmen, als die Bestrahlung einer Rasterposition dauert. Ggf. kann die Bestrahlung der nächsten Rasterpositionen durch Strahlunterbrechung verzögert werden, bis die Berechnungen abgeschlossen sind, falls die Zeit dennoch nicht reicht, um die Berechnung der Dosisänderungs-Beiträge aller nachfolgenden Rasterpositionen zu berechnen oder es wird die Strahl-Intensität vermindert.

c) Ferner vorteilhaft ist es, ein Speicher-Array anzulegen, in dem für jede Rasterposition ein Speicherplatz für den Kompensationswert reserviert ist. Es wird dann der Kompensationswert nicht für jede Rasterposition an dieselbe Stelle im Speicher geschrieben, sondern die Kompensationswerte für jede Rasterposition werden an eine vordefinierte Speicherstelle in dem Array geschrieben. Dies erlaubt, dass für die Bestrahlung der *i*+1-ten Rasterposition eine "0" (das heißt keine Kompensation und daher Anwendung der nominellen Teilchenfluenz) und nicht der Kompensationswert der vorhergehenden Rasterposition *i* gelesen wird, falls der Kompensationswert für die *i*+1-te Rasterposition ausgelesen wird, bevor er in den Speicher geschrieben wurde, z.B. weil die Zeit für die Berechnung nicht ausreichte und zusätzliche Pausen zur Berechnung unerwünscht sind. Vorzugsweise wird das Speicher-Array in einem Kommunikationsspeicher des SKT 72 angelegt.

d) Wenn die akkumulierte Dosisänderung $\Delta D^i$ einer Rasterposition *i* größer oder gleich als die Solldosis $D_{ref}^{ii}$ der Rasterposition *i* ist, entspricht dies einer relativen Dosisänderung $\geq 1$. In diesem Fall hat die Rasterposition *i* schon eine zumindest hinreichende Dosis bei der Bestrahlung der vorherigen Rasterpositionen *k*<*i* erhalten. In diesem Fall kann diese Rasterposition *i* übersprungen werden, wenn dies technisch möglich ist. Dies erfolgt z.B. durch eine kurze Strahlunterbrechung, Fortführung des Behandlungsplans trotz Fehlermeldungen bei Ortsmessung oder dergleichen. Alternativ wird dennoch zumindest eine vordefinierte Mindestdosis angewendet. Die Mindestdosis kann vor der Bestrahlung gewählt werden und beträgt z.B. 1%, 5% oder 30% von $D_{ref}^{ii}$ $\cdot$ Durch die Bestrahlung mit einer Mindestdosis können Inkonsistenzen im Kontrollsystem vermieden werden. Z.B. wird hierdurch sichergestellt, dass auch für diese Rasterposition eine Dosisdeposition vorliegt, mittels welcher mit den Vieldrahtkammern die laterale Strahlposition bestimmt werden kann. Ferner kann durch die Verwendung einer Mindestdosis die Entstehung von abwechselnd schwankenden Dosisverteilungen in den Rasterpositionen verringert werden. Wenn nämlich eine im Vergleich zur Referenzdosis erhöhte Dosis an Rasterposition *i* zu einer Überdosierung der benachbarten Rasterposition *i*+1 führt und die an Rasterposition *i*+1 applizierte Dosis deshalb stark verringert wird, führt dies wiederum zu einer Unterdosierung an der Rasterposition *i*+2 usw. Dieser wechselweise "Aufschaukeleffekt" kann durch die Anwendung der Mindestdosis verringert werden.

e) Wenn an der Beschleunigeranlage das kurzzeitige Unterbrechen der Bestrahlung, z.B. durch so genannte "Knockout-Extraktion" zur Verfügung steht, kann in dem Fall, in dem mehr Rechenzeit bei der Bestrahlung einer Rasterposition benötigt wird, der Strahl, und damit die Bestrahlung, unterbrochen werden.

Mit diesen Maßnahmen kann der zeitliche Ablauf der aufwändigen Berechnung der Kompensationsfaktoren optimiert und die Wahrscheinlichkeit für Fehler im zeitlichen Ablauf gesenkt werden.

Das erfindungsgemäße Verfahren kann für verschiedene Bestrahlungsverfahren angewendet werden, insbesondere:

## 2.a) Ein Bestrahlungsdurchlauf

Die Bestrahlung nach dem Bestrahlungsplan umfasst nur einen Bestrahlungsdurchlauf, d.h. alle Rasterpositionen werden nur einmal abgetastet. Hierbei genügt es, die Werte $D_m^{ik}$ für $i \leq k \leq N$ vor der Bestrahlung zu berechnen, da nach Bestrahlung einer Rasterposition $i$ nur die Teilchenfluenz von Rasterpositionen $k > i$ aktiv verändert werden kann. Insbesondere hierbei wird von distal nach proximal bestrahlt.

## 2.b) Mehrere Bestrahlungsdurchläufe

Die Bestrahlung nach dem Bestrahlungsplan umfasst mehrere Bestrahlungsdurchläufe $1 <= j <= J$, d.h. alle Rasterpositionen werden mehrfach nacheinander abgetastet, ähnlich der klassischen Mehrfachbestrahlung (Rescanning).

Die klassische Mehrfachbestrahlung (Rescanning) wurde bislang allerdings als Alternative zur aktiven Strahlnachführung (Tracking) bei der Bestrahlung bewegter Tumore verwendet und hierbei das Zielvolumen-typischerweise mit erweiterten Sicherheitssäumen - $J$-fach mit jeweils 1/$J$ der geplanten Gesamtdosis bestrahlt, um durch das mehrfache Abtasten eine statistische Mittelung zu erzielen. Erfindungsgemäß berücksichtigen nun vorzugsweise die Kompensationswerte eines Bestrahlungsdurchlaufes $j$ aktiv auch die Dosisänderungen der vorherigen Bestrahlungsdurchläufe. Z.B. wird der Kompensationswert für die $i$-te Rasterposition im $j$-ten Bestrahlungsdurchlauf in Abhängigkeit der Dosisänderungen der $i$-ten Rasterposition bei der Bestrahlung aller Rasterpositionen bei den vorherigen Bestrahlungsdurchläufen $p$ mit $1 <= p < j$ und in Abhängigkeit der Dosisänderungen der $i$-ten Rasterposition bei der Bestrahlung der vorherigen Rasterpositionen $k$ mit $1 <= k < i$ bei dem aktuellen Bestrahlungsdurchlauf $j$ bestimmt.

Somit kann bei dem erfindungsgemäßen Verfahren ggf. bereits bei der ersten Bestrahlung die volle geplante Dosis appliziert werden und in den späteren Bestrahlungen werden lediglich Dosisänderungen auf Grund der Bewegung kompensiert. Jedoch können auch Bruchteile der geplanten Gesamtdosis pro Bestrahlungsdurchlauf angewendet werden. Dies reduziert das Auftreten von Überdosierungen. Es kann sogar noch während der Bestrahlung entschieden werden, ob noch ein weiterer Bestrahlungsdurchlauf vorgenommen wird, oder die bereits deponierte Dosis ausreichend nah an der geplanten Dosis liegt. Bei mehreren Bestrahlungsdurchläufen werden die Werte $D_m^{ik}$ für alle $1 \leq k \leq N$ vor der Bestrahlung berechnet, da ab dem zweiten Bestrahlungsdurchlauf auch Dosisänderungen an Rasterpositionen $k < i$ aktiv verändert werden können.

Das erfindungsgemäße Verfahren kann ferner mit verschiedenen Verfahren zur Bewegungskompensation kombiniert werden. Unter aktiver Bewegungskompensation wird das aktive Nachführen des Strahls an die Bewegung des Zielvolumens (Tracking) verstanden. In Frage kommt z.B. die Kombination mit Folgendem:

## 3.a) Keine aktive Bewegungskompensation.

Das erfindungsgemäße Verfahren kann ggf. die aktive Bewegungskompensation in allen drei Dimensionen ersetzen.

## 3.b) Longitudinale aktive Bewegungskompensation

Es wird eine longitudinale aktive Bewegungskompensation (Nachführen der Energie) ohne laterale aktive Bewegungskompensation durchgeführt.

## 3.c) Laterale aktive Bewegungskompensation

Es wird eine laterale aktive Bewegungskompensation (1- oder 2-dimensional) ohne longitudinale aktive Bewegungskompensation durchgeführt. Dies ist besonders vorteilhaft, da das aktive Nachführen der Strahlenergie relativ aufwändig ist.

Bei nicht vollständiger Bewegungskompensation (Varianten 3.a) bis 3.c) kann sogar das Bragg-Maximum in einer anderen Rasterposition liegen als in der im Bestrahlungsplan vorgesehenen Rasterposition, so dass der Großteil der Dosis an einer "falschen" Rasterposition deponiert wird. Dies wird erfindungsgemäß in den vor der Bestrahlung berechneten $D_m^{ik}$ berücksichtigt. Der hierfür angelegten Tabelle wird während der Bestrahlung entnommen, an welcher Rasterposition der Hauptteil der Dosis deponiert wird. Die kompensierte Dosisänderung dieser Rasterposition wird dann angewendet.

Insbesondere die Variante 3.c. ist klinisch relevant, da hier auf die technisch aufwändige Energiekompensation verzichtet wird und auftretende Dosisänderungen dennoch mit einer Kombination aus lateraler aktiver Bewegungskompensation und dem erfindungsgemäßem rasterpositionsspezifischen Kompensationswert für die Teilchenfluenz kompensiert werden.

3.d) Dreidimensionale aktive Bewegungskompensation

Es wird also ein aktives Nachführen des Strahls an die laterale Bewegung des Zielvolumens in beiden lateralen Dimensionen *x*, *y* und aktives Nachführen der Strahlenergie an die longitudinale Bewegung, z.B. mit einem aktiv gesteuerten Doppelkeilsystem, durchgeführt. In diesem Fall bewirkt das erfindungsgemäße Verfahren eine zusätzliche Verbesserung der Bestrahlung bei einem bewegten Zielvolumen.

Die verschiedenen Möglichkeiten in der Zahl der Bestrahlungsdurchläufe und dem Umfang der aktiven Bewegungskompensation sind untereinander kombinierbar. 3.e) Das erfindungsgemäße Verfahren kann mit Gating kombiniert werden. Beispielsweise wird nur ein Teil der Bewegungsphasen m, z.B. 30% zentriert um die Ausatmung oder es werden alle Phasen außer den Phasen mit unstabiler Tumorbewegung berücksichtigt.

4. Weitere Ausführungsformen

Langfristig, das heißt vor einer Behandlung und für den gesamten Behandlungsverlauf ist die exakte Vorhersage des Tumorbewegungsmusters in einer für die Dosiskompensation notwendigen Genauigkeit nicht möglich. Allerdings können in den Strahlpausen (Spillpausen) verschiedene Verfahren eingesetzt werden, um den kurzfristigen Bewegungsverlauf der Rasterpositionen des Zielvolumens vorherzusagen und in Ansprechen den Bestrahlungsablauf zu verbessern. Für eine Anzahl Rasterpositionen, die voraussichtlich in der Bewegungsphase zu Beginn des nächsten Strahlpakets (Spills) bestrahlt werden, wird unter Berücksichtigung auftretender Dosisänderungen die kompensierte Teilchenfluenz der Rasterpositionen berechnet. Für mehrere Bewegungszyklen kann die Fluenzverteilung in der Regel zwar nicht vorausgesagt werden, da die Übergänge zwischen Bewegungszyklen auf Grund von Bewegungsvariationen nicht exakt vorhergesagt werden können, es kann aber eine Vorhersage der Dosisänderungen wenigstens für einige Rasterpositionen i in Abhängigkeit von möglichen Bewegungsphasen m durchgeführt werden. Es können verschiedene Verfahren genutzt werden, um den Bestrahlungsablauf nach technischen und medizinischen Kriterien zu verbessern.

4.a) Veränderung der Reihenfolge

Es wird die Reihenfolge, in der die Rasterpositionen bestrahlt werden verändert. Zum Beispiel kann in Kombination mit einer innerhalb des Strahlpakets (Spills) veränderten Intensität die Bestrahlungsreihenfolge der Rasterpositionen entsprechend ihrer Teilchenfluenz gewählt werden. Dies ist z.B. bei Knockout-Extraktion möglich.

4.b) Die Verteilung der Teilchenfluenzen für die nächsten Rasterpositionen wird für mehrere Bewegungsphasen *m* vorausberechnet und durch einen Qualitätsindex Q bewertet werden. Entsprechend wird die Bestrahlung erst in der günstigsten Bewegungsphase wieder aufgenommen. Folgende Bewertungskriterien können in den Qualitätsindex Q einfließen:

i) die kompensierten Teilchenfluenzen der Rasterpositionen sind möglichst ähnlich.
ii) die Abweichungen der kompensierten Teilchenfluenzen von den nominellen Teilchenfluenzen aus der Bestrahlungsplanung sind möglichst gering.
iii) Es werden Überdosierungen minimiert. Diesbezüglich kann zwar die Dosis in distalen, d.h. bereits bestrahlten Rasterpositionen nicht mehr aktiv verändert werden, aber für proximale, d.h. später noch zu bestrahlende Rasterpositionen kann durch eine Dosisreduktion eine eventuelle Überdosierung noch reduziert oder gar vermieden werden. Potentielle Überdosierungen können auch mit Gewichtungsfaktoren unterschiedlich gewichtet werden.
iv) Die Zeit bis zum voraussichtlichen Erreichen der zugehörigen Bewegungsphase.

4.c) Gemäß einer bevorzugten Ausführungsform wird die Strahl-Intensität in Ansprechen auf die Teilchenfluenz rasterpositionsabhängig eingestellt. Insbesondere werden Rasterpositionen mit niedriger Teilchenfluenz mit niedriger Intensität und Rasterpositionen mit hoher Teilchenfluenz mit höherer Intensität bestrahlt. Dies führt zu einer Angleichung der Bestrahlungszeiten der Rasterpositionen, wodurch die Dauer der Bestrahlung verringert werden kann. Zusätzlich kann die Angleichung der Bestrahlungszeiten der Rasterpositionen dafür sorgen, dass die Bestrahlung aller Rasterpositionen lange genug dauert, um eine korrekte Systemfunktionalität, wie z.B. die Messung der Strahllage zu gewährleisten und die Zeit, die für die Berechnung der Kompensationswerte zur Verfügung steht, wird ebenfalls nivelliert, wodurch die Gefahr verringert wird, dass der Kompensationswert nicht rechtzeitig berechnet und gespeichert ist.

Die Maßnahmen unter 4.a) bis 4.c) sind miteinander kombinierbar und können jeweils mit den vorstehend unter 1., 2. und 3. beschriebenen Maßnahmen kombiniert werden.

Zusammenfassend können mit der erfindungsgemäßen rasterpositionsabhängigen Echtzeit-Dosiskompensation bei der aktiven Strahlnachführung (Tracking) auftretende Überdosierungen verringert und Unterdosierungen verhindert werden. Die Kombination beider Verfahren lässt bessere Dosisverteilungen als die unterbrochene Bestrahlung (Gating) und klassische Mehrfachbestrahlung (Rescanning) erwarten. Es kann aber ggf. sogar auf die technisch aufwändige Energiekompensation (longitudinale aktive Strahlnachführung) verzichtet werden.


**Patentansprüche**

1. Verfahren zur Steuerung der Dosisapplikation bei der Bestrahlung eines beweglichen Zielvolumens (32) in einem Modell zur Validierung einer Patientenbestrahlung oder einem anderen nichtlebenden Körper (30) mit einem energetischen Strahl (27) mit einem Abtast-Verfahren, wobei

   ein Bestrahlungsplan (46) für einen oder mehrere Bestrahlungsdurchläufe ($j, 1<=j<=J$) mit einer Vielzahl von Rasterpositionen ($i, 1<=i<=N$) des Zielvolumens (32) erstellt wird, wobei in dem Bestrahlungsplan (46) für die Bestrahlung der Rasterpositionen jeweils eine rasterpositionsabhängige nominelle Teilchenfluenz festgelegt wird,

   die Bewegung (33) des Körpers (30) während der Bestrahlung erfasst wird, um die Bewegung der Rasterpositionen während der Bestrahlung zu ermitteln,

   die einzelnen Rasterpositionen mit dem Strahl (27) mit rasterpositionsabhängig gesteuerter Teilchenenergie und Teilchenfluenz nacheinander abgetastet werden, so dass eine rasterpositionsabhängige Dosis an der jeweiligen Rasterposition applizierbar ist, wobei:

   i1) vor der Bestrahlung einer *i*-ten Rasterposition der Vielzahl von Rasterpositionen während des Bestrahlungsdurchlaufes unter Verwendung der Bewegungsdaten diejenige Dosis ermittelt wird, welche die *i*-te Rasterposition bei der Bestrahlung der vorherigen Rasterpositionen ($k, 1<=k<i$) bereits erhalten hat,

   i2) vor der Bestrahlung der *i*-ten Rasterposition in Abhängigkeit von der ermittelten Dosis, welche die *i*-te Rasterposition bei der Bestrahlung der vorherigen Rasterpositionen ($k, 1<=k<i$) bereits erhalten hat, ein Kompensationswert ($\Delta D^i$) für die *i*-te Rasterposition berechnet wird, welcher die im bisherigen Bestrahlungsverlauf akkumulierte Dosisänderung für Rasterposition *i* bezeichnet, die in Echtzeit während der Bestrahlung durch Summation der Einzelbeiträge aller bisher bestrahlter Rasterpositionen zu Rasterposition i berechnet wird,

   i3) vor der Bestrahlung der *i*-ten Rasterposition in Abhängigkeit von dem Kompensationswert ($\Delta D^i$) für die *i*-te Rasterposition und von der nominellen Teilchenfluenz ($F^i_{nom}$) für die *i*-te Rasterposition eine kompensierte Teilchenfluenz ($F^i_{komp}$) für die *i*-te Rasterposition berechnet wird, und wobei

   i4) die *i*-te Rasterposition mit der für die *i*-te Rasterposition ermittelten kompensierten Teilchenfluenz ($F^i_{komp}$) bestrahlt wird,

   wobei die Schritte i1) bis i4) für die Vielzahl der Rasterpositionen ($1<=i<=N$) durchgeführt werden,

   und

   wobei der Kompensationswert für die *i*-te Rasterposition ein mit einer für eine Referenzphase berechneten Referenzdosis ($D^{ii}_{ref}$) für die *i*-te Rasterposition normierter relativer Korrekturfaktor $\dfrac{\Delta D^i}{D^{ii}_{ref}}$ für die *i*-te Rasterposition ist und der Korrekturfaktor auf die im Bestrahlungsplan (46) festgelegte nominelle Teilchenfluenz ($F^i_{nom}$) für die *i*-te Rasterposition angewendet wird, um die kompensierte Teilchenfluenz ($F^i_{komp}$) für die *i*-te Rasterposition zu berechnen.

2. Verfahren nach Anspruch 1,
   wobei die Berechnung des Kompensationswertes ($D^i$) für die *i*-te Rasterposition zumindest teilweise während der Bestrahlung der *i*-1-ten Rasterposition erfolgt.

3. Verfahren nach einem der vorstehenden Ansprüche, wobei für die Berechnung des Kompensationswertes ($\Delta D^i$) für die *i*-te Rasterposition, während der Bestrahlung die Dosisänderungen über alle 1 von 1 bis *i*-1 aufsummiert werden, welche die $i$-te Rasterposition bei der Bestrahlung aller vorher bestrahlter Rasterpositionen 1 erhalten hat und aus der Summe der Dosisänderungen der Kompensationswert für die Bestrahlung der $i$-ten Rasterposition während der Bestrahlung der $i$-1-ten Rasterposition berechnet wird.

4. Verfahren nach einem der vorstehenden Ansprüche, wobei ein Therapiekontrollsystem (70, 72, 84) umfasst ist, welches mit einer Steuereinrichtung (82) für die Beschleunigeranlage (21) kommuniziert und ein Subsystem zur Kontrolle der Teilchenfluenz (72) aufweist, welches die applizierte Teilchenfluenz für alle Rasterpositionen steuert, wobei der Kompensationswert für die $i$-te Rasterposition während der Bestrahlung der $i$-1-ten Rasterposition in einem Speicher des Subsystems zur Kontrolle der Teilchenfluenz (72) gespeichert wird und nachfolgend das Subsystem zur Kontrolle der Teilchenfluenz (72) den Kompensationswert für die $i$-te Rasterposition aus dem Speicher lädt und auf die im Bestrahlungsplan (46) festgelegte nominelle Teilchenfluenz für die $i$-te Rasterposition anwendet, um nachfolgend die $i$-te Rasterposition mit der kompensierten Teilchenfluenz für die $i$-te Rasterposition zu bestrahlen.

5. Verfahren nach einem der vorstehenden Ansprüche, wobei während der Bestrahlung der $i$-ten Rasterposition nicht gleichzeitig die Dosisänderungen zu allen folgenden Rasterpositionen (>=$i$+1), sondern zunächst die Dosisänderung und der Kompensationswert für die $i$+1-te Rasterposition berechnet wird und die Berechnung der Dosisänderungen und der Kompensationswerte für die folgenden Rasterpositionen (>=$i$+2) später erfolgt.

6. Verfahren nach einem der vorstehenden Ansprüche, wobei die rasterpositionsabhängige Verteilung der Teilchenfluenzen für die auf eine Bestrahlungspause folgenden Rasterpositionen für mehrere Bewegungsphasen (m) berechnet und mittels eines Qualitätsindexes (Q) bewertet werden und die Bestrahlung der nächsten Isoenergieschicht nach einer Strahlpause zwischen der Bestrahlung von zwei Isoenergieschichten in Abhängigkeit von dem Qualitätsindex (Q) gestartet wird.

7. Verfahren nach einem der vorstehenden Ansprüche, wobei zumindest eines der folgenden Bewertungskriterien in den Qualitätsindex (Q) einfließt:

    i) Ähnlichkeit der kompensierten Teilchenfluenzen der Rasterpositionen,
    ii) Abweichungen der kompensierten Teilchenfluenzen von den Teilchenfluenzen der Referenzdosen,
    iii) Reduzierung von Überdosierungen der nachfolgenden Rasterpositionen,
    iv) Zeit bis zum voraussichtlichen Erreichen der zugehörigen Bewegungsphase.

8. Bestrahlungsvorrichtung zur Bestrahlung eines beweglichen Zielvolumens in einem Körper mit einem energetischen Strahl (27) mit einem Abtast-Verfahren, insbesondere mit dem Verfahren gemäß einem der vorstehenden Ansprüche, umfassend
eine Bestrahlungsplanungseinrichtung (42, 44) welche eingerichtet ist, um Daten von einem zeitauflösenden Bildgebungssystem (42) zu verarbeiten und anhand dieser Daten einen Bestrahlungsplan (46) für einen oder mehrere Bestrahlungsdurchläufe mit einer Vielzahl von Rasterpositionen (1<=$i$<=$N$) des Zielvolumens (32) zu erstellen und abzuspeichern, wobei der Bestrahlungsplan (46) für die Bestrahlung der Rasterpositionen jeweils einen Referenz-Datensatz mit zumindest Positionsdaten der jeweiligen Rasterposition und einer rasterpositionsabhängigen zu applizierenden nominellen Teilchenfluenz ($F^i_{nom}$) enthält,
eine Bewegungserfassungseinrichtung (58), mit welcher die Bewegung (33) des Körpers (30) während der Bestrahlung erfassbar ist, um die Bewegung der Rasterpositionen während der Bestrahlung zu ermitteln,
ein Therapiekontrollsystem (70, 72, 84), welches eingerichtet ist, die Bestrahlungsvorrichtung so zu steuern, dass die einzelnen Rasterpositionen mit dem Strahl (27) mit rasterpositionsabhängig gesteuerter Teilchenenergie und Teilchenfluenz nacheinander abgetastet werden, so dass eine rasterpositionsabhängige Dosis an der jeweiligen Rasterposition applizierbar ist, und die vor der Bestrahlung abgespeicherten Datensätze des Bestrahlungsplans während der Bestrahlung auszulesen und zu verändern,
wobei das Therapiekontrollsystem (70, 72, 84) ferner eingerichtet ist:

    i1) vor der Bestrahlung einer $i$-ten Rasterposition der Vielzahl von Rasterpositionen während des Bestrahlungsdurchlaufes unter Verwendung der Bewegungsdaten diejenige Dosis zu ermitteln, welche die $i$-te Rasterposition bei der Bestrahlung der vorherigen Rasterpositionen (1<=$k$<$i$) bereits erhalten hat,
    i2) vor der Bestrahlung der $i$-ten Rasterposition in Abhängigkeit von der ermittelten Dosis, welche die $i$-te Rasterposition bei der Bestrahlung der vorherigen Rasterpositionen (1<=$k$<$i$) bereits erhalten hat, einen Kompensationswert für die i-te Rasterposition zu berechnen, welcher die im bisherigen Bestrahlungsverlauf akkumulierte Dosisänderung für Rasterposition i bezeichnet, die in Echtzeit während der Bestrahlung durch Summation der Einzelbeiträge aller bisher bestrahlter Rasterpositionen zu Rasterposition i berechnet wird,

i3) vor der Bestrahlung der *i*-ten Rasterposition in Abhängigkeit von dem Kompensationswert für die *i*-te Rasterposition und von dem im Bestrahlungsplan (46) festgelegten Referenzdatensatz für die *i*-te Rasterposition einen veränderten Datensatz mit kompensierter Teilchenfluenz ($F^i_{komp}$) für die *i*-te Rasterposition zu berechnen, und

i4), die Bestrahlungsvorrichtung so zu steuern, dass die *i*-te Rasterposition mit der berechneten kompensierten Teilchenfluenz ($F^i_{komp}$) bestrahlt wird,

wobei das Therapiekontrollsystem (70, 72, 84) eingerichtet ist, die Schritte i1) bis i4) nacheinander für die Vielzahl der Rasterpositionen (1<=*i*<=*N*) durchzuführen **gekennzeichnet dadurch, dass**
das Therapiekontrollsystem (70, 72, 84) eingerichtet ist, den Kompensationswert für die *i*-te Rasterposition als einen mit einer für eine Referenzphase berechneten Referenzdosis ($D^{ii}_{ref}$) für die *i*-te Rasterposition normierten relativen

Korrekturfaktor $\dfrac{\Delta D^i}{D^{ii}_{ref}}$ für die *i*-te Rasterposition zu berechnen und den Korrekturfaktor auf die im Bestrahlungsplan

festgelegte nominelle Teilchenfluenz ($F^i_{nom}$) für die *i*-te Rasterposition anzuwenden, um die kompensierte Teilchenfluenz ($F^i_{komp}$) für die *i*-te Rasterposition zu berechnen.

**9.** Bestrahlungsvorrichtung nach Anspruch 16,
wobei das Therapiekontrollsystem (70, 72, 84) eingerichtet ist, die Berechnung des Kompensationswertes für die *i*-te Rasterposition zumindest teilweise während der Bestrahlung der *i*-1-ten Rasterposition durchzuführen.

**10.** Bestrahlungsvorrichtung nach einem der vorstehenden Ansprüche,
wobei das Therapiekontrollsystem (70, 72, 84) eingerichtet ist, für die Berechnung des Kompensationswertes für die *i*-te Rasterposition während der Bestrahlung die Dosisänderungen über alle i von 1 bis *i*-1 aufzusummieren, welche die *i*-te Rasterposition bei der Bestrahlung aller vorher bestrahlter Rasterpositionen *1* erhalten hat und aus der Summe der Dosisänderungen den Kompensationswert für die Bestrahlung der *i*-ten Rasterposition während der Bestrahlung der *i*-1-ten Rasterposition zu berechnen.

**11.** Bestrahlungsvorrichtung nach einem der vorstehenden Ansprüche,
wobei das Therapiekontrollsystem (70, 72, 84) ein Subsystem zur Kontrolle der Teilchenfluenz (72) aufweist, welches die applizierte Teilchenfluenz für alle Rasterpositionen steuert und eine Rechteneinrichtung (84), welche den Kompensationswert für die *i*-te Rasterposition während der Bestrahlung der *i*-1-ten Rasterposition berechnet und in einem Speicher des Subsystems zur Kontrolle der Teilchenfluenz (72) speichert, so dass unmittelbar vor der Bestrahlung der *i*-ten Rasterposition der Kompensationswert für die *i*-te Rasterposition für das Subsystem zur Kontrolle der Teilchenfluenz (72) zum Laden aus dem Speicher bereit steht und wobei das Subsystem (72) den geladenen Kompensationswert auf die im Bestrahlungsplan (46) festgelegte nominelle Teilchenfluenz für die *i*-te Rasterposition anwendet, um nachfolgend die *i*-te Rasterposition mit der kompensierten Teilchenfluenz für die *i*-te Rasterposition zu bestrahlen.

**12.** Bestrahlungsvorrichtung nach einem der vorstehenden Ansprüche, wobei das Therapiekontrollsystem (70, 72, 84) eingerichtet ist, während der Bestrahlung der *i*-ten Rasterposition nicht gleichzeitig die Dosisänderungen zu allen folgenden Rasterpositionen, sondern zunächst die Dosisänderung und der Kompensationswert für die *i*+1-te Rasterposition zu berechnen und die Berechnung der Dosisänderungen und der Kompensationswerte für die folgenden Rasterpositionen erst später durchzuführen.

**13.** Bestrahlungsvorrichtung nach einem der vorstehenden Ansprüche, wobei das Therapiekontrollsystem (70, 72, 84) eingerichtet ist, die rasterpositionsabhängige Verteilung der Teilchenfluenzen für die auf eine Strahlpause folgenden Rasterpositionen für mehrere Bewegungsphasen zu berechnen und mittels eines Qualitätsindexes (Q) zu bewerten und die Bestrahlung der nächsten Isoenergieschicht nach einer Strahlpause zwischen der Bestrahlung von zwei Isoenergieschichten in Abhängigkeit von dem Qualitätsindex zu starten.

**14.** Bestrahlungsvorrichtung nach einem der vorstehenden Ansprüche,
wobei zumindest eines der folgenden Bewertungskriterien in den Qualitätsindex einfließt:

i) Ähnlichkeit der kompensierten Teilchenfluenzen der Rasterpositionen,
ii) Abweichungen der kompensierten Teilchenfluenzen von den Teilchenfluenzen der Referenzdosen,
iii) Reduzierung von Überdosierungen der nachfolgenden Rasterpositionen,
iv) Zeit bis zum voraussichtlichen Erreichen der zugehörigen Bewegungsphase.

15. Bestrahlungsvorrichtung nach einem der vorstehenden Ansprüche, wobei das Therapiekontrollsystem (70, 72, 84) eingerichtet ist, den Strahl in zumindest einer Dimension als Funktion der ermittelten Bewegungsdaten der Rasterpositionen des Zielvolumens (32) aktiv der Bewegung der momentan bestrahlten Rasterposition des Zielvolumens (32) nachzuführen, um die Bewegung der momentan bestrahlten Rasterposition des Zielvolumens (32) in der zumindest einen Dimension zu kompensieren.

**Claims**

1. Method to control the dose application in the irradiation of a movable target volume (32) in a model for the validation of a patient irradiation or another non-living body (30) with an energy beam (27) using a scanning method, wherein an irradiation plan (46) is drawn up for one or more irradiation runs ($j, 1 <= j <= J$) with a plurality of grid positions ($i, 1 <= i <= N$) of the target volume (32), wherein

   a nominal particle fluence that is dependent on the grid position is specified in the irradiation plan (46) for the irradiation of the grid positions,

   the movement (33) of the body (30) in the course of irradiation is captured to determine the movement of the grid positions during irradiation,

   the individual grid positions are scanned sequentially with the beam (27) with particle energy and particle fluence that is controlled depending on the grid position, so that a grid position-dependent dose can be delivered to the respective grid position, wherein:

   i1) prior to irradiation of an $i$-th grid position of the plurality of grid positions, that dose which the i-th grid position has already received during irradiation of the previous grid positions ($k, 1 <= k < i$) is established during the irradiation procedure using the movement data,

   i2) prior to irradiation of the $i$-th grid position as a function of the established dose which the $i$-th grid position has already received during irradiation of the previous grid positions ($k, 1 <= k < i$), a compensation value ($\Delta D^i$) is calculated for the $i$-th grid position which characterizes the dose change for the grid position $i$ accumulated in the previous irradiation run, said dose change being calculated in real time in the course of the irradiation of previous grid positions by adding up the individual contributions of all previously irradiated grid positions to grid position $i$,

   i3) prior to irradiation of the $i$-th grid position, as a function of the compensation value ($\Delta D^i$) for the $i$-th grid position and of the nominal particle fluence ($F^i_{nom}$) for the i-th grid position, a compensated particle fluence ($F^i_{comp}$) is calculated for the $i$-th grid position, and wherein

   i4) the $i$-th grid position is irradiated with the compensated particle fluence ($F^i_{comp}$) determined for the $i$-th grid position,

   wherein steps i1) to i4) are carried out for the plurality of the grid positions ($1 <= i <= N$), and

   wherein the compensation value for the $i$-th grid position is a relative correction factor $\frac{\Delta D^i}{D^{ii}_{ref}}$ normalized for the $i$-th grid position with a reference dose ($D^{ii}_{ref}$), calculated for a reference phase and wherein the correction factor is applied to the nominal particle fluence ($F^i_{nom}$) specified in the irradiation plan (46) for the $i$-th grid position in order to calculate the compensated particle fluence ($F^i_{comp}$) for the $i$-th grid position.

2. Method according to claim 1, wherein the calculation of the compensation value ($D^i$) for the $i$-th grid position is carried out at least in part during irradiation of the $i$-1-th grid position.

3. Method according to one of the above claims, wherein for the calculation of the compensation value ($\Delta D^i$) for the $i$-th grid position, the dose changes which the $i$-th grid position has received during irradiation of all previously irradiated grid positions 1 are summated during irradiation over all the 1's from 1 to $i$-1, and wherein the compensation value for the irradiation of the $i$-th grid position is calculated from the sum of the dose changes during irradiation of the $i$-1-th grid position.

4. Method according to one of the above claims, wherein a therapy monitoring system (70, 72, 84) is included which communicates with a control device (82) for the acceleration system (21) and which has a subsystem to control the particle fluence (72) which controls the applied particle fluence for all grid positions,

wherein the compensation value for the $i$-th grid position during irradiation of the $i$-1-th grid position is stored in a memory of the subsystem for control of the particle fluence (72), and subsequently the subsystem for control of the particle fluence loads the compensation value for the $i$-th grid position from the memory and applies it to the nominal particle fluence for the $i$-th grid position specified in the irradiation plan (46) in order to subsequently irradiate the $i$-th grid position with the compensated particle fluence for the i-th grid position.

5. Method according to one of the above claims,

wherein during irradiation of the $i$-th grid position not all dose changes for all following grid positions ($>=i+1$) are calculated at the same time, but initially the dose change and the compensation value for the $i+1$-th grid position is calculated and the calculation of the dose changes and the compensation values for the subsequent grid positions ($>=i+2$) is carried out later on.

6. Method according to one of the above claims, wherein the grid position-dependent distribution of the particle fluences for the grid positions following an irradiation pause is calculated for several movement phases (m) and is evaluated using a quality index (Q), and the irradiation of the next isoenergy layer is started as a function of the quality index (Q) after an irradiation pause between the irradiations of two isoenergy layers.

7. Method according to one of the above claims, wherein at least one of the following evaluation criteria flows into the quality index (Q):

   i) similarity between the compensated particle fluences of the grid positions,
   ii) deviations between the compensated particle fluences and the particle fluences of the reference doses,
   iii) reduction of the overdoses for the following grid positions,
   iv) time to the anticipated attainment of the associated movement phase.

8. Irradiation device for the irradiation of a movable target volume in a body with an energy beam (27) using a scanning method, in particular the method according to one of the above claims, comprising

an irradiation planning device (42, 44) which is set up to process data from a time-resolving imaging system (42) and on the basis of these data to prepare and store an irradiation plan (46) for one or more irradiation runs with a plurality of grid positions ($1<=i<=N$) of the target volume (32), wherein the irradiation plan (46) for the irradiation of the grid positions in each case contains a reference data set with at least position data of the respective grid position and a grid position-dependent nominal particle fluence ($F^i_{nom}$) to be applied,

a movement capture device (58), with which the movement (33) of the body (30) can be captured during irradiation, in order to establish the movement of the grid positions during irradiation,

a therapy monitoring system (70, 72, 84), which is set up to control the irradiation device in such a way that the individual grid positions are scanned sequentially with the beam (27) with grid position-dependently controlled particle energy and particle fluence, so that a grid position-dependent dose can be applied to the respective grid position, and the data sets of the irradiation plan stored prior to irradiation can be read out and changed during irradiation, wherein the therapy monitoring system (70, 72, 84) is further set up

i1) to establish, prior to the irradiation of an i-th grid position of the plurality of grid positions during the irradiation run, and using the movement data that dose which the $i$-th grid position has already received during irradiation of the previous grid positions ($1<=k<i$),

i2) to calculate, prior to irradiation of the $i$-th grid position, as a function of the established dose which the $i$-th grid position has already received during irradiation of the previous grid positions ($1<=k<i$), a compensation value for the $i$-th grid position which characterizes the dose change for grid position i accumulated in the previous irradiation run, which dose change is calculated during irradiation in real time by summation of the individual contributions of all grid positions irradiated so far to grid position $i$,

i3) to calculate, prior to irradiation of the $i$-th grid position, a modified data set with compensated particle fluence ($F^i_{comp}$) for the $i$-th grid position as a function of the compensation value for the $i$-th grid position and of the reference data set for the $i$-th grid position specified in the irradiation plan (46), and

i4) to control the irradiation device such that the $i$-th grid position is irradiated with the calculated compensated particle fluence ($F^i c_{omp}$),

wherein the therapy monitoring system (70, 72, 84) is set up so that the steps i1) to i4) are carried out sequentially for the plurality of grid positions ($1<=i<=N$), **characterized in that**

the therapy monitoring system (70, 72, 84) is set up to calculate the compensation value for the $i$-th grid position as a relative correction factor $\frac{\Delta D^i}{D^{ii}_{ref}}$ normalized for the $i$-th grid position with a reference dose ($D^{ii}_{ref}$) calculated for a reference phase, and to apply the correction factor to the nominal particle fluence ($F^i_{nom}$) specified in the irradiation plan for the i-th grid position in order to calculate the compensated particle fluence ($F^i_{comp}$) for the i-th grid position.

9. Irradiation device according to claim 16, wherein the therapy monitoring system (70, 72, 84) is set up so that the calculation of the compensation value for the $i$-th grid position is carried out at least in part during irradiation of the $i$-1-th grid position.

10. Irradiation device according to one of the above claims, wherein the therapy monitoring system (70, 72, 84) is set up, for the calculation of the compensation value for the $i$-th grid position during irradiation, to summate all dose changes over all the i's from 1 to i-1 which the $i$-th grid position has received during irradiation of all previously irradiated grid positions 1 and to calculate from the sum of the dose changes the compensation value for the irradiation of the $i$-th grid position during irradiation of the $i$-1-th grid position.

11. Irradiation device according to one of the above claims, wherein the therapy monitoring system (70, 72, 84) has a subsystem to control particle fluence (72) which controls the applied particle fluence for all grid positions and a calculation device (84) which calculates the compensation value for the $i$-th grid position during irradiation of the $i$-1-th grid position and stores it in a memory of the subsystem to control particle fluence (72), so that immediately prior to irradiation of the $i$-th grid position the compensation value for the $i$-th grid position for the subsystem to control the particle fluence (72) is ready to be loaded out of the memory and wherein the subsystem (72) applies the loaded compensation value to the nominal particle fluence specified in the irradiation plan (46) for the $i$-th grid position in order to subsequently irradiate the $i$-th grid position with the compensated particle fluence for the $i$-th grid position.

12. Irradiation device according to one of the above claims, wherein the therapy monitoring system (70, 72, 84) is set up such that during irradiation of the $i$-th grid position there is no calculation at the same time of the dose changes for all following grid positions, but instead the dose change and the compensation value for the $i$+1-th grid position are calculated and the calculation of the dose changes and of the compensation values for the following grid positions are carried out later on.

13. Irradiation device according to one of the above claims, wherein the therapy monitoring system (70, 72, 84) is set up so that the grid position-dependent distribution of particle fluences is calculated for the grid positions following an irradiation pause for several movement phases and is evaluated using a quality index (Q), and the irradiation of the next isoenergy layer is started after an irradiation pause between the irradiations of two isoenergy layers as a function of the quality index.

14. Irradiation device according to one of the above claims, wherein at least one of the following evaluation criteria flows into the quality index:

    i) similarity between the compensated particle fluences of the grid positions,
    ii) deviations between the compensated particle fluences and the particle fluences of the reference doses
    iii) reduction of the overdoses for the following grid positions,
    iv) time to the anticipated attainment of the associated movement phase.

15. Irradiation device according to one of the above claims, wherein the therapy monitoring system (70, 72, 84) is set up so that the beam actively tracks the movement of the grid position of the target volume (32) currently being irradiated, in at least one dimension as a function of the established movement data, in order to compensate for the movement of the grid position of the target volume (32) currently being irradiated in the at least one dimension.

## Revendications

1. Procédé destiné à contrôler l'application d'une dose lors de l'irradiation d'un volume cible (32) mobile dans un modèle afin de valider à l'aide d'un procédé de balayage une irradiation d'un patient ou d'un autre corps non vivant (30) avec un faisceau énergétique (27), sachant
qu'est établi un plan d'irradiation (46) pour un ou plusieurs cycle(s) d'irradiation ($j$,1<=$j$<=$J$) avec une pluralité de positions de trame ($i$,$1<=i<= N$) du volume cible (32), et que dans le plan d'irradiation (46) pour l'irradiation des positions de trame est à chaque fois définie une fluence de particules nominale en fonction des positions de trame, que le mouvement (33) du corps (30) est enregistré pendant l'irradiation afin de déterminer le mouvement des positions de trame pendant l'irradiation,

que les différentes positions de trame sont successivement balayées au moyen du faisceau (27) avec une énergie des particules et une fluence de particules réglées en fonction des positions de trame, de sorte qu'une dose dépendant de la position de trame peut être appliquée aux positions de trame respectives, sachant que :

i1) avant l'irradiation d'une $i^{ème}$ position de trame de la pluralité de positions de trame est déterminée, en utilisant les données de mouvement pendant le cycle d'irradiation, la dose déjà reçue par la $i^{ème}$ position de trame lors de l'irradiation de la position de trame précédente ($k$,$1<=k<i$),

i2) avant l'irradiation de la $i^{ème}$ position de trame, en fonction de la dose déterminée que la $i^{ème}$ position de trame a déjà reçue lors de l'irradiation de la position de trame précédente ($k$,$1<=k<i$), est calculée pour la $i^{ème}$ position de trame une valeur compensatoire ($\Delta D^i$) qui caractérise le changement de dose accumulé au cours des irradiations précédentes de la position de trame $i$ et ce changement de dose est calculé en temps réel pendant l'irradiation en additionnant toutes les contributions individuelles à la position de trame $i$ apportées par les autres positions de trame irradiées auparavant,

i3) avant l'irradiation de la $i^{ème}$ position de trame, en fonction de la valeur compensatoire ($\Delta D^i$) pour la $i^{ème}$ position de trame et de la fluence de particules nominale ($F^i_{nom}$) pour la $i^{ème}$ position de trame, est calculée une fluence de particules compensée ($F^i_{komp}$) pour la $i^{ème}$ position de trame, et que

i4) la $i^{ème}$ position de trame est irradiée avec la fluence de particules compensée ($F^i_{komp}$) déterminée pour la $i^{ème}$ position de trame,

sachant que les étapes i1) à i4) sont réalisées pour la pluralité des positions de trame ($l<=i<=N$), et que

la valeur compensatoire pour la $i^{ème}$ position de trame est un coefficient de correction relatif $\frac{\Delta D^i}{D^{ii}_{ref}}$ normalisé pour la $i^{ème}$ position de trame avec une dose de référence ($D^{ii}_{ref}$) calculée pour une phase de référence pour la $i^{ème}$ position de trame, et que ce coefficient de correction est appliqué à la fluence de particules nominale ($F^i_{nom}$) définie dans le plan d'irradiation (46) pour la $i^{ème}$ position de trame afin de calculer la fluence de particules compensée ($F^i_{komp}$) pour cette $i^{ème}$ position de trame.

**2.** Procédé selon la revendication 1,
dans lequel le calcul de la valeur compensatoire ($D^i$) pour la $i^{ème}$ position de trame a lieu au moins en partie pendant l'irradiation de la ($i$-1)$^{ème}$ position de trame.

**3.** Procédé selon l'une des revendications précédentes, dans lequel pour le calcul de la valeur compensatoire ($\Delta D^i$) pour la $i^{ème}$ position de trame, sont additionnés pendant l'irradiation les changements de dose de tous les 1 de 1 jusqu'à $i$-1 que la $i^{ème}$ position de trame a reçu lors de l'irradiation de toutes les positions de trame 1 irradiés auparavant, et à partir de la somme des changements de dose, la valeur compensatoire pour l'irradiation de la $i^{ème}$ position de trame est calculée pendant l'irradiation de la ($i$-1)$^{ème}$ position de trame.

**4.** Procédé selon l'une des revendications précédentes, dans lequel est compris un système de contrôle de thérapie (70, 72, 84) qui communique avec un dispositif de commande (82) pour l'accélérateur (21) et présente un sous-système pour le contrôle de la fluence de particules (72), lequel régule la fluence de particules appliquée à toutes les positions de trame,
sachant que pendant l'irradiation de la ($i$-1)$^{ème}$ position de trame, la valeur compensatoire pour la $i^{ème}$ position de trame est stockée dans une mémoire du sous-systèmes pour contrôler la fluence de particules (72), et qu'ensuite pour contrôler la fluence de particules (72), le sous-système charge depuis la mémoire la valeur compensatoire pour la $i^{ème}$ position de trame et utilise la fluence de particules nominale pour la $i^{ème}$ position de trame définie dans le plan d'irradiation (46) afin d'irradier ensuite la $i^{ème}$ position de trame avec la fluence de particules compensée pour la $i^{ème}$ position de trame.

**5.** Procédé selon l'une des revendications précédentes, dans
lequel pendant l'irradiation de la $i^{ème}$ position de trame ne sont pas calculés simultanément les changements de dose pour toutes les positions de trame suivantes ($>=i+1$), mais que sont tout d'abord calculés le changement de dose et la valeur compensatoire pour la ($i+1$)$^{ème}$ position de trame, et que le calcul des changements de dose et des valeurs compensatoires pour les positions de trame suivantes ($>=i+2$) a lieu plus tard.

**6.** Procédé selon l'une des revendications précédentes, dans lequel la distribution en fonction des positions de trame des fluences de particules pour les positions de trame succédant à une pause d'irradiation est calculée pour plusieurs phases de mouvement (m) et évaluée au moyen d'un indice de qualité (Q), et que l'irradiation de la prochaine couche isoénergétique après une pause d'irradiation entre l'irradiation de deux couches isoénergétiques est lancée en fonction de cet indice de qualité (Q).

**7.** Procédé selon l'une des revendications précédentes, dans lequel l'indice de qualité (Q) prend en compte au moins l'un des critères d'évaluation suivants :

    i) similitude des fluences de particules compensées des positions de trame,
    ii) écarts des fluences de particules compensées par rapport aux fluences de particules des doses de référence,
    iii) réduction de surdosages pour les positions de trame suivantes,
    iv) durée jusqu'à l'atteinte prévue de la phase de mouvement associée.

**8.** Dispositif d'irradiation destiné à irradier un volume cible mobile dans un corps avec un faisceau énergétique (27), intégrant un procédé de balayage, en particulier le procédé selon l'une des revendications précédentes, comprenant un dispositif d'élaboration de plans d'irradiation (42, 44) qui est conçu pour traiter des données d'un système d'imagerie (42) à pouvoir de résolution dans le temps, et à l'aide de ces données, élaborer et enregistrer un plan d'irradiation (46) pour un ou plusieurs cycle(s) d'irradiation avec une pluralité de positions de trame ($1<=i<=N$) du volume cible (32), sachant que le plan d'irradiation (46) pour l'irradiation des positions de trame comprend respectivement un jeu de données de référence avec au moins des données de position des positions de trame respectives et une fluence de particules nominale ($F^i_{nom}$) à appliquer en fonction des positions de trame, un dispositif de détection de mouvement (58), avec lequel peut être détecté le mouvement (33) du corps (30) pendant l'irradiation afin de déterminer le mouvement des positions de trame au cours de l'irradiation, un système de contrôle de thérapie (70, 72, 84) qui est conçu pour diriger le dispositif d'irradiation de manière telle que les différentes positions de trame sont successivement balayées par le faisceau (27) avec l'énergie des particules et la fluence de particules réglées en fonction des positions de trame, de sorte qu'une dose en fonction des positions de trame est applicable aux positions de trame respectives, et pour extraire et modifier pendant l'irradiation les jeux de données du plan d'irradiation enregistrées avant l'irradiation, sachant que le système de contrôle de thérapie (70, 72, 84) est en outre conçu :

    i1) pour déterminer avant l'irradiation d'une
    $i^{ème}$ position de trame de la pluralité de positions de trame, en utilisant les données de mouvement pendant le cycle d'irradiation, la dose déjà reçue par la $i^{ème}$ position de trame lors de l'irradiation de la position de trame précédente ($1<=k<i$),
    i2) pour calculer avant l'irradiation de la $i^{ème}$ position de trame, en fonction de la dose déterminée que la $i^{ème}$ position de trame a déjà reçue lors de l'irradiation de la position de trame précédente ($1<=k<i$), une valeur compensatoire pour la $i^{ème}$ position de trame, qui caractérise le changement de dose accumulé au cours des irradiations précédentes de la position de trame i et qui est calculée en temps réel pendant l'irradiation en additionnant toutes les contributions individuelles à la position de trame $i$ apportées par les autres positions de trame irradiées auparavant,
    i3) pour calculer avant l'irradiation de la $i^{ème}$ position de trame, en fonction de la valeur compensatoire pour la $i^{ème}$ position de trame et du jeu de données de référence défini dans le plan d'irradiation (46) pour la $i^{ème}$ position de trame, un jeu de données modifié avec une fluence de particules compensée ($F^i_{komp}$) pour la $i^{ème}$ position de trame, et
    i4) pour diriger le dispositif d'irradiation de manière telle que la $i^{ème}$ position de trame est irradiée avec la fluence de particules compensée ($F^i_{komp}$) calculée,

sachant que le système de contrôle de thérapie (70, 72, 84) est conçu pour réaliser successivement les étapes i1) à i4) pour la pluralité des positions de trame ($1<=i<=N$), **caractérisé en ce que**

le système de contrôle de thérapie (70, 72, 84) est conçu pour calculer la valeur compensatoire pour la $i^{ème}$ position de trame en tant que coefficient de correction relatif $\frac{\Delta D^i}{D^{ii}_{ref}}$ normalisé pour la $i^{ème}$ position de trame avec une dose de référence ($D^{ii}_{ref}$) calculée pour une phase de référence pour la $i^{ème}$ position de trame, et utiliser ce coefficient de correction sur la fluence de particules nominale ($F^i_{nom}$) définie dans le plan d'irradiation pour la $i^{ème}$ position de trame afin de calculer la fluence de particules compensée ($F^i_{komp}$) pour cette $i^{ème}$ position de trame.

**9.** Dispositif d'irradiation selon la revendication 16, dans lequel le système de contrôle de thérapie (70, 72, 84) est conçu pour effectuer le calcul de la valeur compensatoire pour la $i^{ème}$ position de trame au moins en partie pendant l'irradiation de la $(i\text{-}1)^{ème}$ position de trame.

**10.** Dispositif d'irradiation selon l'une des revendications précédentes, dans lequel pour le calcul de la valeur compensatoire pour la $i^{ème}$ position de trame, le système de contrôle de thérapie (70, 72, 84) est conçu pour additionner pendant l'irradiation les changements de dose de tous les $i$ de 1 jusqu'à $i\text{-}1$ que la $i^{ème}$ position de trame a reçu lors de l'irradiation de toutes les positions de trame 1 irradiés auparavant, et pour calculer à partir de la somme des changements la valeur compensatoire pour l'irradiation de la $i^{ème}$ position de trame pendant l'irradiation de la $(i\text{-}1)^{ème}$ position de trame.

**11.** Dispositif d'irradiation selon l'une des revendications précédentes, sachant que le système de contrôle de thérapie (70, 72, 84) présente un sous-système pour le contrôle de la fluence de particules (72), lequel régule la fluence de particules appliquée pour toutes les positions de trame, ainsi qu'une unité de calcul (84), laquelle calcule la valeur compensatoire pour la $i^{ème}$ position de trame pendant l'irradiation de la $(i\text{-}1)^{ème}$ position de trame et la stocke dans une mémoire du sous-systèmes pour contrôler la fluence de particules (72), de sorte que directement avant l'irradiation de la $i^{ème}$ position de trame, la valeur compensatoire pour la $i^{ème}$ position de trame est prête à être extraite de la mémoire pour le sous-système de contrôle de la fluence de particules (72), et sachant que le sous-système (72) utilise la valeur compensatoire extraite sur la fluence de particules nominale définie dans le plan d'irradiation (46) pour la $i^{ème}$ position de trame, afin d'irradier ensuite la $i^{ème}$ position de trame avec la fluence de particules compensée pour la $i^{ème}$ position de trame.

**12.** Dispositif d'irradiation selon l'une des revendications précédentes, dans lequel le système de contrôle de thérapie (70, 72, 84) est conçu pour ne pas calculer simultanément les changements de dose pour toutes les positions de trame suivantes pendant l'irradiation de la $i^{ème}$ position de trame, mais pour calculer tout d'abord le changement de dose et la valeur compensatoire pour la $(i\text{+}1)^{ème}$ position de trame, et effectuer plus tard le calcul des changements de dose et des valeurs compensatoires pour les positions de trame suivantes.

**13.** Dispositif d'irradiation selon l'une des revendications précédentes, dans lequel le système de contrôle de thérapie (70, 72, 84) est conçu pour calculer pour plusieurs phases de mouvement la distribution en fonction des positions de trame des fluences de particules pour les positions de trame succédant à une pause d'irradiation et l'évaluer au moyen d'un indice de qualité (Q), et pour lancer en fonction de l'indice de qualité l'irradiation de la prochaine couche isoénergétique après une pause d'irradiation entre l'irradiation de deux couches isoénergétiques.

**14.** Dispositif d'irradiation selon l'une des revendications précédentes, dans lequel l'indice de qualité (Q) prend en compte au moins l'un des critères d'évaluation suivants :

i) similitude des fluences de particules compensées des positions de trame,
ii) écarts des fluences de particules compensées par rapport aux fluences de particules des doses de référence,
iii) réduction de surdosages pour les positions de trame suivantes,
iv) durée jusqu'à l'atteinte prévue de la phase de mouvement associée.

**15.** Dispositif d'irradiation selon l'une des revendications précédentes, dans lequel le système de contrôle de thérapie (70, 72, 84) est conçu pour guider activement le faisceau dans au moins une dimension en fonction des données de mouvement déterminées pour les positions de trame du volume cible (32), afin de suivre le mouvement de la position de trame du volume cible (32) momentanément irradiée, de façon à compenser dans l'au moins une dimension le mouvement de la position de trame momentanément irradiée du volume cible (32).

254 Me V/u C-12
14

300 Me V/u C-12
16

12

135 Me V Protonen
18

18 MV Photonen

Tiefe in Wasser / cm

Fig. 1

Fig. 2

Fig. 3

Fig. 4

Fig. 5

Fig. 6 a

Fig. 6 b

Fig. 6 c

Fig. 7

| Bewegungserfassung m(i) | | Vorberechnete Datenbasis zur Dosiskompensation $D_m^{ik}$ |

Recheneinrichtung zur Berechnung des Kompensationswertes

1.  $\Delta d_{m(i)}^{(i)(i+l)}$    wird aus Datenbasis berechnet

2.  $\dfrac{\Delta D^{i+l}}{D_{ref}^{(i+l)(i+l)}}$    wird aus Kommunikationsspeicher geschrieben

3.  $\Delta d_{m(i)}^{(i)(k)}$    werden für $(i+2) \leq k \leq n$ aus Datenbasis berechnet

Letzte Rasterposition der Isoenergieschicht → n

Subsystem zur Kontrolle der Teilchenfluenz SKT)

1.  $\dfrac{\Delta D^{i}}{D_{ref}^{ii}}$  wird ausgelesen

2.  nominelle Teilchenfluenz wird entsprechend verändert:

$$F_{komp}^{i} = F_{nom}^{i}\left(l - \frac{\Delta D^{i}}{D_{ref}^{(i)(i)}}\right)$$

| 1 | 2 | 3 | ... | i | i+n | ... | n | ... | N |

Kommunikationsspeicher des SKT

Letzte Rasterposition des Bestrahlungsplans → N

Fig. 8

Fig. 9

EP 2 504 061 B1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102005063220 A1
- DE 102007045879

**In der Beschreibung aufgeführte Nicht-Patentliteratur**

- **S.O. GRÖTZINGER ; TU DARMSTADT.** *Volume Conformal Irradiation of Moving Target Volumes with scanned ion beams,* 2004
- **C. BERT.** *Bestrahlungsplanung für bewegte Zielvolumina in der Tumortherapie mit gescanntem Kohlenstoffstrahl,* 2006
- **BERT C. ; RIETZEL E.** 4D treatment planning for scanned ion beams. *Radiat. Oncol.,* 2007, vol. 2, 24
- **KRÄMER et al.** Treatment planning for heavy-ion radiotherapy: physical beam model and dose optimization. *Phys Med Biol,* 2000, vol. 45, 33299-3317
- **KRÄMER et al.** Treatment planning for heavy-ion radiotherapy: calculation and optimization of biologically effective dose. *Phys Med Biol,* 2000, vol. 45, 3319-3330
- **JÄKEL ; KRÄMER.** Treatment planning for heavy ion irradiation. *Phys. Med.,* vol. 14, 53-62